# EUROPEAN PATENT APPLICATION

(11) **EP 2 371 951 A1**
(43) Date of publication of application: **05.10.2011**
(21) Application number: 09829167.7
(22) Date of filing: 27.11.2009
(51) Int. Cl.: C12N 15/00, C12N 1/15, C12N 1/19, C12N 1/21, C12N 5/10, C12N 15/09, C12P 21/02, C12Q 1/48, C12Q 1/68

(54) **NOVEL MutS PROTEIN AND METHOD OF USING SAME TO DETERMINE MUTATIONS**

(30) Priority: 27.11.2008 JP 2008302573
(71) Applicant: Riken, Wako-shi, Saitama 351-0198 (JP); Kabushiki Kaisha Dnaform, Kanagawa 230-0046 (JP)
(72) Inventor: HAYASHIZAKI, Yoshihide, Yokokama-shi, Kanagawa 230-0045 (JP); ITOH, Masayoshi, Yokokama-shi, Kanagawa 230-0045 (JP); USUI, Kengo, Yokokama-shi, Kanagawa 230-0045 (JP); MOMURA, Kazuhito, Yokokama-shi, Kanagawa 230-0045 (JP); KANAMORI, Hajime, Yokokama-shi, Kanagawa 230-0046 (JP)
(74) Representative: Woods, Geoffrey Corlett
(86) International application number: PCT/JP2009/070051
(87) International publication number: WO 2010/061922

(57) **Abstract**

A method for determining the presence or absence of a mutation on the basis of the presence or absence of amplification with high reliability is provided. A target sequence including a target site contained in a sample nucleic acid is amplified using a primer that can hybridize to a region including the target site contained in the sample nucleic acid in the presence of a novel MutS having an amino acid sequence of SEQ ID NO: 2, and then the presence or absence of a mutation at the target site is determined on the basis of the presence or absence of amplification. The novel MutS binds more specifically to a mismatched base pair than to a fully-matched base pair, whereby an extension reaction caused by a mismatch-binding primer is suppressed. Thus, according to the present invention, the presence or absence of a mutation can be determined with high reliability.

## Description

### Technical Field

The present invention relates to a novel MutS protein and a method for determining a mutation using the same.

### Background Art

As a method for diagnosing, treating, and preventing various diseases, detection of a gene mutation has been conducted recently. The gene mutation is deeply involved in morbidity, drug metabolizing ability, and the like, whereby the detection of the gene mutation has great significance in medical care.

As a method for detecting a gene mutation, for example, a method in which a target sequence including a target site at which a mutation to be detected occurs in a target gene is amplified, and the presence or absence of a mutation is determined on the basis of the presence or absence of amplification has been developed. In this method, for example, a primer that can hybridize to a region including the target site is used. For example, in the case where a primer has a sequence that is completely complementary to a sequence including the target site of a mutant type, when amplification is found, it can be determined that the target gene is of a mutant type, assuming that the target sequence has been amplified because the primer has been annealed to the target gene including the target site of a mutant type. On the other hand, for example, in the case where a primer has a sequence that is completely complementary to a sequence including the target site of a wild type, when amplification is found, it can be determined that the target gene is of a wild type, assuming that the target sequence has been amplified because the primer has been annealed to the target gene including the target site of a wild type.

However, in such a method, even when the primer is not complementary to a template, there is a case that the primer anneals to the template, so that the target sequence is amplified. That is, for example, there is a problem in that when a primer that is completely complementary to a sequence including the target site of a mutant type is used as mentioned above, the primer is annealed to a template including the target site of a mutant type, so that a wild-type target sequence is amplified. Thus, the accuracy of mutation detection is reduced.

Hence, in order to prevent such a problem from occurring, a method using a mismatch binding protein what is called a MutS protein or the like has been proposed. The mismatch binding protein generally recognizes and binds to a mismatched base pair contained in a double-stranded nucleic acid. In the case where the above-mentioned detection of a gene mutation is conducted in the presence of this mismatch binding protein, even when the primer forms a mismatched base pair, the mismatch binding protein binds to the mismatched base pair. Thus, an extension from the primer is suppressed. Therefore, a reduction in the accuracy of mutation detection is preventable (see Patent Document 1). As such a mismatch binding protein, for example, a Taq MutS protein derived from *Thermus aquaticus* is used.

### Prior Art Document

### Patent Document

Patent Document 1: Japanese Patent No. 3942627

### Summary of Invention

### Problem to be Solved by the Invention

However, in order to achieve further increases in detection accuracy, it is desired that a mismatch binding protein can recognize and bind further specifically to a mismatched base pair. Hence the present invention is intended to provide a new mismatch binding protein that can recognize and bind specifically to a mismatched base pair and a method for determining a mutation using the same with high reliability.

### Means for Solving Problem

In order to achieve the aforementioned object, the novel MutS protein of the present invention includes: an amino acid sequence (A) or (B) below:
(A) an amino acid sequence shown in SEQ ID NO: 2; and
(B) an amino acid sequence that is obtained by deletion, displacement, insertion, or addition of one or several amino acids in the amino acid sequence (A) and is of a protein having a binding activity to a mismatched base pair contained in a double-stranded nucleic acid.

The determination method of the present invention is a determination method for determining a presence or absence of a mutation at a target site in a sample nucleic acid. The determination method includes the step (I) or (I'), and the step (II) below:
(I) the step of amplifying a target sequence including the target site contained in the sample nucleic acid using a primer that can hybridize to a region including the target site contained in the sample nucleic acid in the presence of the novel MutS protein of the present invention:
   (I') the step of amplifying a target sequence including the target site contained in the sample nucleic acid using a primer for amplifying the sample nucleic acid in the presence of the novel MutS protein of the present invention and a probe that can hybridize to the region including the target site contained in the sample nucleic acid;
(II) the step of checking for presence or absence of amplification.

### Effects of the Invention

As the results of earnest studies, the inventors of the present invention obtained the novel MutS protein derived from genus *Alicyclohacillus* through cloning a novel gene of a MutS protein derived from genus *Alicyclobacillus.* Hereinafter, the novel MutS protein is referred to as "Aac MutS". The Aac MutS of the present invention can specifically recognize and bind to a double-stranded nucleic acid having a mismatched base pair, for example. Therefore, when the Aac MutS of the present invention is used for amplification of a target sequence including a target site, the Aac MutS binds specifically to a mismatched base pair, so that an extension caused by a primer can be effectively suppressed. Thus, according to the determination method of the present invention using the Aac MutS of the present invention, the presence or absence of a mutation can be determined on the basis of the presence or absence of amplification with high accuracy. Therefore, it can be said that the Aac MutS and the determination method of the present invention are very useful tools in the fields of gene analyses, for example.

### Brief Description of Drawings

[FIG. 1] FIG. 1 shows graphs each indicating the result obtained by conducting a nucleic acid binding assay in the presence of Aac MutS in Example 2 of the present invention.
[FIG. 2] FIG. 2 shows graphs each indicating the result obtained by conducting a nucleic acid binding assay in the presence of Aac MutS and ADP in Example 3-1 of the present invention.
[FIG. 3] FIG. 3 shows graphs each indicating the result obtained by conducting a nucleic acid binding assay in the presence of Aac MutS and ATP in Example 3-2 of the present invention.
[FIG. 4] FIG. 4 shows electrophoretograms each indicating the result obtained by conducting a gel shift assay in the presence of Aac MutS in Example 4 of the present invention.
[FIG. 5] FIG. 5 shows graphs each indicating an amplification profile obtained by conducting an isothermal amplification reaction in the presence of Aac MutS in Example 5 of the present invention.
[FIG. 6] FIG. 6 shows graphs each indicating an amplification profile obtained by conducting an isothermal amplification reaction in the presence of Taq MutS in Comparative Example 4.
[FIG. 7] FIG. 7 shows graphs each indicating an amplification profile obtained by conducting an isothermal amplification reaction in the presence ofAac MutS and Taq MutS in Example 6-1 of the present invention.
[FIG. 8] FIG. 8 shows graphs each indicating an amplification profile obtained by conducting an isothermal amplification reaction in the presence ofAac MutS and Taq MutS in Example 6-2 of the present invention.
[FIG. 9] FIG. 9 is a schematic diagram showing the mechanism of action of nucleic acid synthesis using a first primer in the Smart Amplification Process method.
[FIG. 10] FIG. 10 is a schematic diagram showing an example of a second primer used for the Smart Amplification Process method.
[FIG. 11] FIG. 11 is a schematic diagram showing the mechanism of action of the Smart Amplification Process method.
[FIG. 12] FIG. 12 is a schematic diagram showing the mechanism of action of the Smart Amplification Process method.

### Description of Embodiments

### <Aac MutS>

As mentioned above, the Aac MutS of the present invention includes an amino acid sequence (A) or (B) below:
(A) an amino acid sequence shown in SEQ ID NO: 2; and
(B) an amino acid sequence that is obtained by deletion, displacement, insertion, or addition of one or several amino acids in the amino acid sequence (A) and is of a protein having a binding activity to a mismatched base pair contained in a double-stranded nucleic acid.

The MutS protein (hereinafter referred to as "MutS") also is referred to as a mismatch binding protein or a mismatch recognition protein, for example. The MutS can generally recognize and bind to a mismatched base pair contained in a double-stranded nucleic acid. In the present invention, the "mismatched base pair" means not a normal pair of bases that are complementary to each other such as a combination of adenine and thymine or uracil or a combination of guanine and cytosine but a pair of bases that are not complementary to each other. Further, the "mismatched base pair" also includes the meaning that, in a double-stranded nucleic acid, a base in one strand has been deleted at a site corresponding to the predetermined base in the other strand, so that a base pair at the site has been deleted.
Hereinafter, the double-stranded nucleic acid having a mismatched base pair is referred to as a "mismatched double strand or a hetero double strand", and a binding by which a mismatched base pair is formed is referred to as a "mismatch binding". In the present invention, the mismatched double strand is, for example, substantially complementary to each other, and means a double strand including regions that are not complementary to each other because of having one or more mismatched base pairs. On the other hand, in contrast to the mismatched base pair, hereinafter, a pair of bases that are completely complementary to each other is referred to as a "fully-matched base pair", a double-stranded nucleic acid in which strands are completely complementary to each other is referred to as a "fully-matched double strand", and a binding between bases that are completely complementary to each other is referred to as a "full match binding".

As mentioned above, the Aac MutS of the present invention has a binding activity to a mismatched base pair contained in a double-stranded nucleic acid. Further, the Aac MutS of the present invention is, for example, preferably in any of the situations below. The Aac MutS of the present invention does not have a binding activity to a double-stranded nucleic acid (fully-matched double-stranded nucleic acid) including a pair of bases that are completely complementary to each other. The binding activity to the fully-matched double-stranded nucleic acid is a detection limit thereof or less. The binding activity to the fully-matched double-stranded nucleic acid is 1/1.25 or less (4/5 or less) of the binding activity to the mismatched double-stranded nucleic acid. More preferably, the binding activity to the fully-matched double-stranded nucleic acid is 1/4 or less, 1/120 or less, 1/200 or less, or 1/205 or less of the same.

The Aac MutS of the present invention can be isolated from, for example, bacteria of genus *Alicyclohacillus,* preferably *Alicyclobacillus acidocaldazius,* and more preferably *Alicyclobacillus acidocaldarius* subsp. *Acidocaldazius* JCM5260. This strain can be, for example, purchased from RIKEN (independent administrative institution) BioResource Center, Japan Collection of Microorganisms (http://www.jcm.riken.jp/JCM/Ordering J. shtml). The Aac MutS of the present invention also can be produced by gene-engineering techniques using an Aac MutS gene.

As shown in the amino acid sequence (B), the Aac MutS of the present invention includes an amino acid sequence that is obtained by deletion, displacement, insertion, or addition of one or several amino acids in the amino acid sequence shown in SEQ ID NO: 2 and contains a protein having a binding activity to a mismatched base pair contained in a double-stranded nucleic acid, for example. The "several amino acids" refers, for example, to the number of amino acid residues that is about 5% to 10% of the number of full-length amino acid residues, and is, for example, about 1 to 86, preferably about 1 to 43, more preferably about 1 to 21, and most preferably about 1 to 10.

The Aac MutS of the present invention includes an amino acid sequence having a homology of 50% or more to a protein having the amino acid sequence (A), preferably having a homology of 70% or more, 80% or more, 85% or more, 90% or more, 97% or more, or 98% or more to the same and contains a protein having a binding activity to a mismatched base pair contained in a double-stranded nucleic acid, for example. Usually, the homology of protein can be indicated as a percentage of the identity between amino acid sequences of the two proteins when they are aligned appropriately, and generally, it means the occurrence ratio of a perfect match between both amino acid sequences. The appropriate alignment between both the sequences for comparison of the identity can be decided using various algorithms, for example, a BLAST algorithm (Altschul SF J Mol Biol 1990 Oct 5; 215(3): 403-10).

A method for measuring an activity of the MutS is not limited, and it can be measured by various methods well known to those skilled in the art. Specifically, for example, the method described in documents of the Journal of Biological Chemistry 276, 34339-34347, 2001; doi: 10.1074/jbc. M104256200 can be used.

The molecular weight of the Aac MutS of the present invention is, for example, in the range from 86,000 to 105,500 Da, and preferably from 91,000 to 100,800 Da. The molecular weight of the Aac MutS having an amino acid sequence shown in SEQ ID NO: 2 is 95,984 Da.

As chemical characteristics of the Aac MutS of the present invention, for example, it is superior in stability until the temperature thereof reaches specifically 65°C, the optimum temperature thereof is, for example, in the range from about 50°C to about 60°C, and the optimum pH thereof is, for example in the range from 7 to 9.

In the present invention, for example, general methods based on molecular biology, microbiology, a genetic transformation technology, and the like can be conducted with reference to standard reference books in the art. Examples of these books include documents below:
- Molecular Cloning: A Laboratory Manual, Third Edition (Sambrook & Russell, Cold Spring Harbor Laboratory Press, 2001);
- Current Protocols in Molecular biology (edited by Ausubel et al., John Wiley & Sons, 1987);
- A series of Methods in Enzymology (Academic Press); PCR Protocols: Methods in Molecular Biology (edited by Bartlett & Stirling, Humana Press, 2003); and
- Antibodies: A Laboratory Manual (edited by Harlow & Lane, Cold Spring Harbor Laboratory Press, 1987).

Furthermore, reagents, kits, and the like referred to herein are available from commercial vendors such as, for example, Sigma, Aldrich, Invitrogen/GIBCO, Clontech, Stratagene, Qiagen, Promega, Roche Diagnostics, Becton-Dickinson, and Takara Bio Inc.

### <Aac MutS gene>

The novel nucleic acid of the present invention is a nucleic acid that encodes the novel MutS of the present invention. The novel nucleic acid includes: any of nucleic acids (a) to (f) below:
(a) a nucleic acid having a base sequence of SEQ ID NO: 1;
(b) a nucleic acid that hybridizes to the nucleic acid (a) under stringent conditions and encodes a protein having a binding activity to a mismatched base pair contained in a double-stranded nucleic acid;
(c) a nucleic acid that has a base sequence having a homology of 80% or more to a base sequence of the nucleic acid (a) and encodes a protein having a binding activity to a mismatched base pair contained in a double-stranded nucleic acid;
(d) a nucleic acid that has a base sequence obtained by deletion, displacement, insertion, or addition of one or several bases in the base sequence of the nucleic acid (a) and encodes a protein having a binding activity to a mismatched base pair contained in a double-stranded nucleic acid;
(e) a nucleic acid that encodes a protein having an amino acid sequence shown in SEQ ID NO: 2; and
(f) a nucleic acid that has an amino acid sequence obtained by deletion, displacement, insertion, or addition of one or several amino acids in the amino acid sequence shown in SEQ ID NO: 2 and encodes a protein having a binding activity to a mismatched base pair contained in a double-stranded nucleic acid.

Hereinafter, the novel nucleic acid of the present invention is referred to as "Aac MutS gene". The Aac MutS gene of the present invention includes the meaning of any of the nucleic acids (b) to (f) other than the meaning of the nucleic acid (a) having a base sequence shown in SEQ ID NO: 1. Examples of the Aac MutS gene of the present invention include a degenerate variant of the base sequence of the nucleic acid (a) to (f) and a nucleic acid that has a base sequence that is complementary to that of the nucleic acid (a) to (f) and encodes a protein having a binding activity to a mismatched base pair contained in a double-stranded nucleic acid. Examples of the Aac MutS gene of the present invention also include, in addition to DNAs, RNAs (mRNAs) to the DNAs.

In the nucleic acid (b), "hybridization under stringent conditions" means hybridization under experimental conditions well known to those skilled in the art, for example. Specifically, the "stringent conditions" refers to conditions under which a hybrid formed can be identified after conducting hybridization at 60°C to 68°C in the presence of 0.7 to 1 mol/l NaCl and then washing at 65°C to 68°C using a 0.1- to 2-fold SSC solution. Note here that 1 x SSC is composed of 150 mmol/L NaCl and 15 mmol/L sodium citrate. In order to select the stringency, for example, the salt concentration and the temperature in the washing step can be optimized as appropriate. Furthermore, it is common general technical knowledge in the art to add, for example, formamide, SDS, or the like in order to improve the stringency.

In the nucleic acid (c), the homology is, for example, 80% or more, preferably 90% or more, and more preferably 95% or more. The homology can be calculated using the BLAST or the like under default conditions, for example.

In the nucleic acid (d), the "several bases" refers to, for example, the number of bases that is about 10% to 20% of the number of the total bases in a base sequence shown in SEQ ID NO: 1, and is, for example, about 1 to about 520, preferably about 1 to about 260, more preferably about 1 to about 130, and most preferably about 1 to about 65.

In the nucleic acid (f), the "amino acid sequence obtained by deletion, displacement, insertion, or addition of one or several amino acids" means the same as the explanation for the Aac MutS of the present invention, for example.

The Aac MutS gene of the present invention may be, for example, extracted from bacterial cells of genus *Alicyclobacillus* or synthesized by gene-engineering techniques or chemical techniques as mentioned above.

### <Recombinant vector>

As mentioned above, the recombinant vector of the present invention includes the Aac MutS gene of the present invention. It is only necessary that the recombinant vector of the present invention includes the Aac MutS gene of the present invention, and configurations other than this are not at all limited.

The recombinant vector of the present invention can be obtained by ligating (inserting) the Aac MutS gene of the present invention to a suitable vector, for example. The vector to which the Aac MutS gene of the present invention is inserted is not particularly limited, as long as, for example, it can be replicated in a host, and examples thereof include plasmid DNA and phage DNA. Examples of the plasmid DNA include: plasmids derived from *Escherichia coli* such as pBR322, pBR325, pUC118, and pUC119; plasmids derived from *Bacillus subtilis* such as pUB110 and pTP5; and plasmids derived from yeast such as YEp13, YEp24, and YCp50. Examples of the phage DNA include λ phage such as Charon 4A, Charon 21A, EMBL3, EMBL4, λgt10, λgt11, and λZAP. Further, it also is possible to use an animal virus such as retrovirus or vaccinia virus, an insect virus vector such as baculovirus, or the like.

A method for inserting the Aac MutS gene of the present invention into a vector is not particularly limited, and a conventionally known method can be employed. Specific examples thereof include a method in which, for example, a purified Aac MutS gene (DNA) is cleaved with a suitable restriction enzyme and the resultant DNA fragment is inserted into a restriction enzyme site or a multicloning site of a suitable vector DNA, thus ligating between both the DNA fragment and the vector, and the like. It is preferred that the Aac MutS gene of the present invention is incorporated into the vector under the condition under which a protein encoded by the gene is expressed, for example. Thus, for example, not only a promoter such as a trp promoter, a lac promoter, a PL promoter, or a tac promoter but also a cis element such as an enhancer, a splicing signal, a poly-A addition signal, a selection marker, a ribosome binding sequence (an SD sequence, a KOZAK sequence, or the like), or the like may be ligated to the vector, if desired. Examples of the selection marker include drug resistance genes such as a dihydrofolate reductase gene, an ampicillin resistance gene, and a neomycin resistance gene.

### <Transformant>

As mentioned above, the transformant of the present invention includes the recombinant vector of the present invention. It is only necessary that the transformant of the present invention includes the recombinant vector of the present invention, and configurations other than this are not at all limited.

The transformant of the present invention is obtained by, for example, introducing the recombinant vector of the present invention into a host. The host is not particularly limited as long as it can express the Aac MutS of the present invention by the recombinant vector of the present invention and can be selected according to, for example, the type of the recombinant vector with consideration given to a host-vector system. Specific examples of the host include bacterium belonging to: genus *Escherichia* such as *Escherichia coli;* genus *Bacillus* such as *Bacillus subtilis;* genus *Pseudomonas* such as *Pseudomonas putida;* and genus *Rhizobium* such as *Rhizobium meliloti* and the like. In addition, yeast such as *Saccharomyces cerevisiae* or *Schizosaccharomyces pombe,* an animal cell such as a COS cell or CHO cell, or an insect cell such as Sf9 or Sf21 can be used. A method for conducting the transformation is not particularly limited, and a conventionally known method can be employed. Specific examples thereof include a method using a calcium ion (Cohen, S. N. et al. (1972) Proc. Natl. Acad. Sci., USA 69, 2110-2114), a DEAE dextran method, and an electroporation method.

### < Aac MutS production method>

The Aac MutS of the present invention can be prepared by, for example, culturing the transformant of the present invention. The Aac MutS production method of the present invention includes culturing the transformant of the present invention as mentioned above, for example. The Aac MutS production method of the present invention may further include isolating a Aac MutS protein from an obtained culture solution, for example. The "culture" means, for example, not only a culture solution containing the cultured transformant but also a supernatant of the culture solution, cultured cells or cultured bacterial cells, or a homogenate thereof. Furthermore, "a method for culturing the transformant of the present invention" is conducted in accordance with, for example, a usual method applied to the culture of the host, and the conditions and the like thereof can be decided as appropriate according to the type of the host and the like, for example.

In the case where the Aac MutS of the present invention is produced in the bacterial cells or the cells, it can be isolated by, for example, homogenizing the bacterial cells or the cells after the culturing. On the other hand, in the case where the Aac MutS of the present invention is produced outside the bacterial cells or the cells, the culture solution is used as it is, or it can be isolated by removing the bacterial cells or the cells from the culture solution through centrifugation or the like, for example. After the isolation, the Aac MutS of the present invention can be purified from the culture by using a biochemical method commonly used for isolation and purification of proteins either alone or, if necessary, in combination with another method. The purification method is not particularly limited, and examples thereof include ammonium sulfate precipitation, gel chromatography, ion exchange chromatography, and affinity chromatography. Furthermore, for example, in the case where a protein having a tag sequence was expressed for purification, the tag sequence can be removed by a protease treatment or the like during or after the purifying step.

### < Method for determining mutation>

The method for determining a mutation of the present invention can be a first determination method or a second determination method shown below.

As mentioned above, the first determination method for determining a mutation of the present invention is for determining the presence or absence of a mutation at a target site in a sample nucleic acid. The first determination method includes the steps (I) and (II) below:
(I) the step of amplifying a target sequence including the target site contained in the sample nucleic acid using a primer that can hybridize to a region including the target site contained in the sample nucleic acid in a presence of the novel MutS of the present invention;
(II) the step of checking the presence or absence of amplification.

In the present invention, hereinafter, a nucleic acid sequence in which a base at a target site is standard such as a nucleic acid sequence in which the target site is of a standard genotype (a normal type or a wild type) is referred to as a "normal-type sequence or wild-type sequence". On the other hand, a nucleic acid sequence in which a base at a target site is different from that at the same contained in the normal-type sequence is referred to as a "mutant-type sequence". The "target site" means a specific site indicating a base that is different between the wild-type sequence and the mutant-type sequence, and may represent a single base or a sequence of double bases or more. The "sample nucleic acid" means a nucleic acid to be subjected to a determination of the presence or absence of a mutation at the target site, i.e., a nucleic acid to be subjected to a determination of whether the target site therein is of a wild type or a mutant type or a determination of whether or not a sequence thereof except for the target site is the same as that of a wild-type sequence. The sample nucleic acid may include not only a nucleic acid contained in a sample to be subjected to the determination method of the present invention and a nucleic acid at the start of an amplification reaction but also a nucleic acid synthesized by the amplification reaction. It is also referred to as a "template nucleic acid". The "target sequence" includes the meaning of, for example, not only a nucleic acid sequence to be amplified, being contained in the sample nucleic acid but also a sequence having a nucleic acid sequence to be amplified or a nucleic acid sequence that is complementary thereto. Moreover, the primer can hybridize (anneal) to a region including a target site, whereby it is also referred to as a "target primer". The "region including a target site" can hybridize to the target primer, whereby, hereinafter, it also is referred to as a hybrid region. In the present invention, "mutation" may be any of displacement, deletion, addition, and insertion.

As mentioned above, the Aac MutS used for the determination method of the present invention can recognize and bind specifically to a mismatched base pair. For example, it has higher specificity to a mismatched base pair than that to a pair of bases that are complementary to each other, i.e., fully-matched base pair. Therefore, according to the first determination method of the present invention, when a target primer that can hybridize to a region including the target site contained in the sample nucleic acid binds to the sample nucleic acid with a mismatch, the Aac MutS binds specifically to a mismatched site, whereby an extension reaction caused by the target primer is specifically suppressed. This can result in avoiding wrong amplification caused by the mismatch-binding target primer. Thus, the presence or absence of a mutation can be determined on the basis of the presence or absence of amplification with high reliability.

The determination method of the present invention is useful in the determination of susceptibility to various diseases, whether or not the diseases are developed, and sensitivity and a resistance to medicines for the diseases. For example, when the susceptibility to the diseases is determined on the basis of the presence or absence of a mutation at a target site contained in a target gene, a sequence obtained from a healthy subject is a normal-type sequence, and a sequence obtained from a patient with the diseases is a mutant-type sequence. Further, a gene obtained from a subject is used as a sample nucleic acid, and whether or not a target site is of a normal type or a mutant type is determined. Accordingly, when the target site is of a normal type, it can be determined that the sample nucleic acid has a normal-type sequence, and the subject has a low risk of developing the diseases or is a healthy subject. On the other hand, when the target site is of a mutant type, it can be determined that the sample nucleic acid has a mutant-type sequence, and the subject has a high risk of developing the diseases or is a subject with the diseases.

The target site in the sample nucleic acid may be single base (mononucleotide) or double bases (dinucleotide) or more. In the latter case, the bases may be consecutive or nonconsecutive. Among them, it is preferred that the determination method of the present invention is applied in the case where the target site to be determined is single base or mononucleotide. When there is a difference in only a single base between the target primer and a hybrid region in the sample nucleic acid, a sequence of the target primer except for the single base is completely homologous to that in the hybrid region. Thus, even when the sample nucleic acid has a base that is a mismatch to that of a target primer, the target primer hybridizes easily to the sample nucleic acid. However, according to the Aac MutS of the present invention, for example, even when there is a difference in only a single base between the sample nucleic acid and a target primer, and the target primer hybridizes to the sample nucleic acid, the Aac MutS binds specifically to a mismatched base pair, whereby a wrong extension reaction can be suppressed. Thus, the determination method of the present invention is suitable for a determination of single nucleotide polymorphism.

A mismatch generated when the primer hybridizes to the sample nucleic acid is, for example, a mismatch in a single base, mismatches in plural bases that are consecutive, or mismatches in plural bases that are nonconsecutive. The upper limit of the number of the plural bases is not particularly limited, and is, for example, preferably the number with which the state of being a double strand between the sample nucleic acid and the primer can be maintained. Specifically, the upper limit is, for example, five bases or less, more preferably three bases or less, and particularly preferably two bases or less even though it depends on the lengths (the numbers of bases) of both strands hybridizing to each other.

A target primer that can hybridize to the region in which a base at the target site is of a mutant type can be used as a primer for amplification in the step (I) of the first determination method of the present invention, for example. With the primer, when amplification is found in the step (II), it can be determined that the base at the target site is of a mutant type, and when amplification is not found in the same, it can be determined that the base at the target site is of a normal type. On the other hand, a target primer that can hybridize to the region in which the base at the target site is of a normal type also can be used as the primer for amplification in the step (I), for example. With the primer, when amplification is found in the step (II), it can be determined that the base at the target site is of a normal type, and when amplification is not found in the same, it can be determined that the base at the target site is of a mutant type.

Conventionally, in order to suppress an extension reaction caused by a mismatch-binding primer, for example, MutS such as Taq MutS has been used. However, the conventional MutS has low substrate specificity. Thus, there are problems in that the MutS binds to not only a mismatched double strand but also a fully-matched double strand, and even when the MutS binds to the mismatched double strand, the MutS is dissociated easily from the mismatched double strand. Further, there is a risk that desired amplification cannot be checked. In contrast, the Aac MutS of the present invention has high substrate specificity to a mismatched double strand, for example. Thus, a binding of the Aac MutS to a fully-matched double strand can be suppressed as compared with a binding of conventional MutS to the same. Moreover, the Aac MutS is difficult to be dissociated from the mismatched double strand. Thus, it becomes possible to conduct a determination with high reliability.

The amount of the Aac MutS to be added to a reaction solution used for the amplification reaction is not particularly limited and can be decided as appropriate according to, for example, the amounts of the sample nucleic acid at the start of the reaction and the various primers. Specific examples thereof are as follows (hereinafter the same applies). The amount of the sample nucleic acid at the start of the reaction is, for example, in the range from 0.1 to 1000 ng, preferably from 0.5 to 500 ng, and more preferably from 1 to 100 ng, per 25 µL of the reaction solution. The total amount of primers is, for example, in the range from 0.01 to 1000 µmol, preferably from 0.05 to 500 µmol, and more preferably from 0.1 to 100 µmol, per the same. The amount of the Aac MutS is, for example, in the range from 0.01 to 1000 µg, preferably from 0.05 to 500 µg, and more preferably from 0.1 to 100 µg, per the same.

In the first determination method of the present invention, it is preferred that the target sequence is amplified in the co-presentof the Aac MutS and at least one additive selected from the group consisting of additives ofADP (adenosine 5'-diphosphate), ATP (adenosine 5'-triphosphate), and derivatives thereof. By conducting the nucleic acid amplification in the presence of the additive, the binding rate of the Aac MutS of the present invention to a mismatched base pair can be increased, for example. By amplifying the target sequence in the co-presence of the Aac MutS and the ADP or the derivative thereof among them, dissociation of the binding between the mismatched base pair and the Aac MutS of the present invention also can be suppressed. Therefore, an extension reaction caused by a target primer with which a mismatched base pair is formed can be suppressed further effectively. Thus, a determination result of a mutation with higher reliability can be obtained. Examples of the derivatives include ATP-γ-S (adenosine 5'-O-(3-thio triphosphate)) andAMP-PNP (adenosine 5'-[ß,yimide] triphosphate). The additives may be used alone or in a combination of two or more of them, for example. The additive contains preferably ADP or the derivative thereof, and more preferably ADP.

The amount of the additive to be added to the reaction solution used for the amplification reaction is not particularly limited and can be decided as appropriate according to, for example, the amounts of the Aac MutS, the sample nucleic acid at the start of the reaction, and the various primers. Specific examples thereof are as follows. The concentration of the additive in the reaction solution is, for example, preferably in the range from 0.01 to 100 mmol/L, more preferably from 0.05 to 50 mmol/L, and particularly preferably from 0.1 to 10mmol/L. In this case, the concentrations of the Aac MutS and the like in the reaction solution are preferably in the above-mentioned ranges.

In the first determination method of the present invention, it is preferred that the target sequence is amplified in the coexistence of the Aac MutS of the present invention and MutS except for one derived from genus *Alicyclobacillus.* The MutS except for one derived from genus *Alicyclobacillus* can be, for example, MutS derived from genus *Thermus,* and specifically from *Thermus aquaticus* (hereinafter referred to as "Taq MutS"). MutS derived from genus *Bacillus* or the like can also be used.

The Aac MutS of the present invention can be used in combination with other MutS, for example. With the combined used of the Aac MutS of the present invention and the other MutS, for example, the total amount of MutS can be reduced as compared with the amount of Aac MutS in the case of using the Aac MutS alone. Further, the effective concentration range can be expanded as compared with that in the case of using conventional Taq MutS or the like alone. Specific examples are as follows. Preferably, the amount of the Aac MutS is in the range from 0.01 to 1000 µg, the amount of the other MutS is in the range from 0.01 to 1000 µg, and the total amount of the Aac MutS and the other MutS is in the range from 0.02 to 2000 µg, per 25 µL of the reaction solution. More preferably, the amount of the Aac MutS is in the range from 0.05 to 500 µg, the amount of the other MutS is in the range from 0.05 to 500 µg, and the total amount of the Aac MutS and the other MutS is in the range from 0.1 to 1000 µg, per the same. Particularly preferably, the amount of the Aac MutS is in the range from 0.1 to 100 µg, the amount of the other MutS is in the range from 0.1 to 100 µg, and the total amount of the Aac MutS and the other MutS is in the range from 0.2 to 200 µg, per the same. In this case, the amounts of the sample nucleic acid at the start of the reaction and the like in the reaction solution are preferably in the above-mentioned ranges. The ratio of the other MutS (T) to be added with respect to the Aac MutS (A) (weight ratio (A: T)) is, for example, preferably in the range from 1 : 0.05 to 1 : 50, more preferably from 1 : 0.25 to 1 : 25, and particularly preferably from 1 : 0.5 to 1 : 5.

The Aac MutS and the other MutS may be activated by an activator in order further to prevent it from binding to a fully-matched double-stranded nucleic acid, for example. The activator is not particularly limited, and examples thereof include ATP, ADP, ATP-γ-S (adenosine 5'-O-(3-thio triphosphate)), and AMP-PNP (adenosine 5'-[β,γ-imide] triphosphate), and in addition, nucleotides that can bind to MutS. The activation can be conducted by incubating the MutS and the activator at room temperature for several seconds to several minutes.

In the first determination method of the present invention, the target sequence also may be amplified in the coexistence of the Aac MutS and the single-strand binding protein (SSB). With the combined use of the Aac MutS and the SSB, the binding of the Aac MutS of the present invention to the single-stranded nucleic acid can yet further be prevented. The SSB is not particularly limited, and a conventionally known protein can be used. Specific examples of the SSB include single-strand binding proteins derived from *Escherichia coli, Drosophila,* and *Xenopus laevis,* gene 32 protein derived from T4 Bacteriophage, and in addition, these proteins derived from other species.

In the present invention, the type of the sample nucleic acid at the start of the reaction is not at all limited, and it may be, for example, a nucleic acid derived from a natural product or a non-natural product that is obtained by synthesis or the like. Examples of the sample nucleic acid include polynucleotides such as DNA and RNA. Note here that the polynucleotide includes oligonucleotide. The polynucleotide may contain an unmodified nucleotide or a modified nucleotide or may contain a natural nucleotide or a non-natural nucleotide, for example. The non-natural nucleotide contains bases other than the bases contained in the natural nucleotide, and examples of the bases include xanthosines, diaminopyrimidines, isoG, and isoC (Proc. Natl. Acad. Sci. USA 92, 6329-6333, 1995). The polynucleotide may contain artificially synthesized nucleic acid such as LNA, PNA (peptide nucleic acid), morpholino nucleic acid, methylphosphonate nucleic acid, or S-oligo nucleic acid or any of chimeric molecules thereof. Examples of the DNA include genomic DNA, cDNA, and synthesized DNA. Examples of the RNA include total RNA, mRNA, rRNA, siRNA, hnRNA, synthesized RNA, spliced RNA, and unspliced RNA. When the sample nucleic acid is RNA, for example, it may be possible that DNA (cDNA) is generated from RNA by a reverse transcription reaction, and then, an amplification reaction is conducted using DNA thus obtained as a template. The sample nucleic acid at the start of the reaction can be prepared from, for example, a sample derived from a biological body, such as blood, an organ, a tissue, or a cell or a sample containing microorganisms such as food, a soil, or drainage. Examples of the biological body include animals including human and nonhuman and plants. Examples of the RNA contained in a sample include RNA that is present in nucleus, cytoplasm, or the like and RNA derived from infected virus and infected bacterium. Collecting a sample nucleic acid from a sample is not particularly limited, and a conventionally known method can be employed. A collected nucleic acid can be purified or fragmented if required.

In the first determination method of the present invention, the sample nucleic acid may be, for example, a double-stranded nucleic acid or a single-stranded nucleic acid. The double-stranded nucleic acid may be any of a double-stranded DNA, a double-stranded RNA, a double strand between DNA and RNA. The double-stranded nucleic acid as it is may be used as a template nucleic acid. For example, it is also possible to use one obtained by amplifying the double-stranded nucleic acid by a vector such as phage or plasmid as a template nucleic acid. When sample nucleic acid is a double-stranded nucleic acid, an amplification reaction may be started using the double-stranded nucleic acid as it is, or it may include a step of degenerating the double-stranded nucleic acid into single-stranded nucleic acid. The degeneration method is not particularly limited, and examples thereof include a method in which the temperature of a reaction solution is changed and a method in which the pH of a reaction solution is changed. In the former case, it is preferred that a double-stranded nucleic acid is degenerated into single-stranded nucleic acid by increasing the temperature to 40°C to 120°C, preferably to about 95°C, and subsequently, a primer is annealed to the single-stranded nucleic acids by decreasing the temperature to 0°C to 65°C. In the latter case, it is preferred that a double-stranded nucleic acid is degenerated into single-stranded nucleic acid by increasing the pH to about 7 to about 14, and subsequently, a primer is annealed to the single-stranded nucleic acid by decreasing the pH to about 6 to about 9.

The type of a primer used for the first determination method of the present invention is not particularly limited and can be decided as appropriate according to, for example, the types of a sample nucleic acid, a target sequence, and a nucleic acid amplification method and the like. When two or more types of primers are used for the first determination method of the present invention, for example, at least one of them is preferably a target primer that can hybridize to a region including the target site contained in the sample nucleic acid as mentioned above. It is preferred that a primer pair set composed of a primer that hybridizes to a sense strand and a primer that hybridizes to an antisense strand is used as a primer for amplifying the predetermined target sequence. One type of the primer pair set may be used, or two or more types thereof may be used in combination. The primer pair set and the other primers may be used in combination. In the first determination method of the present invention, two or more types of target sequences may be amplified in the same reaction solution. In this case, it is preferred that at least one type of target primer that can hybridize to a region including the target site for each target sequence is used as a primer for amplifying each target sequence.

The primer used for the present invention is not particularly limited and can be decided as appropriate according to, for example, the types of a sample nucleic acid, a target sequence, a nucleic acid amplification method described below, and the like. The primer may be a polynucleotide derived from a natural product or a non-natural product obtained by synthesis or the like. The polynucleotide may be, for example, any of deoxyribonucleotide, modified deoxyribonucleotide, ribonucleotide, modified ribonucleotide, polynucleotides containing derivatives thereof, and chimeric polynucleotides containing the same. The derivative of the ribonucleotide can be, for example, ribonucleotide with a sulfur atom substituted for an oxygen atom at the α-position. The primer further may contain an artificially synthesized nucleic acid such as LNA, PNA (peptide nucleic acid), morpholino nucleic acid, methylphosphonate nucleic acid, or S-oligo nucleic acid or chimerapolynucleotide thereof. The polynucleotide includes the meaning of olugonucleotide.

In the present invention, for example, a primer preferably hybridizes (anneals) to the predetermined region (hybrid region) contained in the sample nucleic acid under stringent conditions, and more preferably hybridizes to only the predetermined region under stringent conditions. The stringent conditions can be decided depending on, for example, the melting temperature Tm (°C) of a double strand between a primer and a strand complementary thereto and the salt concentration of a hybridization solution. Reference can be made to J. Sambrook, E. F. Frisch, T. Maniatis; Molecular Cloning 2nd edition, Cold Spring Harbor Laboratory (1989), for example. For example, specifically, when hybridization between the sample nucleic acid and a primer is conducted at a slightly lower temperature than the melting temperature of the primer, the primer can hybridize specifically to the predetermined region. Such a primer can be designed using, for example, commercially available primer construction software such as Primer 3 (produced by Whitehead Institute for Biomedical Research).

In the first determination method of the present invention, a polymerase generally can be used for amplifying the target sequence. The polymerase is not particularly limited, and a conventionally known polymerase can be used. The polymerase may be a naturally-derived polymerase, an enzyme obtained by gene-engineering techniques, or a variant in which a mutation has been added artificially. Specific examples of the polymerase include polymerases derived from genus *Alicyclobacillus,* genus *Thermus,* genus *Bacillus,* genus *Geobacillus,* and *Escherichia coli.* The polymerase derived from *Alicyclobacillus* is preferably a polymerase derived from *Alicyclobacillus acidocaldarius,* and is specifically a polymerase derived from *Alicyclobacillus acidocaldarius* subsp. *Acidocaldarius* JCM5260, for example. Examples of the polymerase derived from genus *Thermus* include a DNA polymerase derived from *Thermus aquaticus* (Taq DNApolymerase) and a DNA polymerase derived from *Thermus thermophilus* (Tth DNA polymerase). The polymerase derived from genus *Bacillus* is, for example, preferably a polymerase derived from thermophilic genus *Bacillus,* and the specific examples thereof include a DNA polymerase derived from *Bacillus stearothermophilus* (Bst DNA polymerase) and a DNA polymerase derived from *Bacillus caldotenax* (Bca DNA polymerase: registered trademark). Examples of the Bca DNA polymerase include a BcaBEST DNA polymerase and a Bca (exo-) DNA polymerase. The polymerase derived from genus *Geobacillus* is, for example, preferably a polymerase derived from *Geobacillus caldoxylosilyticus* and can be a polymerase derived from *Geobacillus caldoxylosilyticus* DSM12041 as a specific example. In addition, examples of the polymerase include a Vent (registered trademark) DNA polymerase ,a Vent (registered trademark) (Exo-) DNA polymerase, a DeepVent (registered trademark) DNA polymerase, a Deep Vent (registered trademark) (Exo-) DNA polymerase, a Φ29 phage DNA polymerase, a MS-2 phage DNA polymerase, a Z-Taq DNA polymerase, a Pfu DNA polymerase, a Pfu turbo DNA polymerase, a KOD DNA polymerase, a 9°Nm DNA polymerase, and a Therminator DNA polymerase. When the template nucleic acid contains a non-natural nucleotide as mentioned above, from the viewpoint of, for example, incorporation efficiency, it is preferred that a Y188L/E478Q mutant type HIV I reverse transcriptase, an AMV reverse transcriptase, a Klenow fragment of a DNA polymerase, a 9° Nm DNA polymerase, a HotTub DNA polymerase, or the like is used (Michael Sismour. 1 et al., Biochemistry, 42, No.28, 8598, 2003, the specification of U.S. Pat. No. 6617106, Michael J. Lutz et al., Bioorganic & Medical Chemistry letters 8, 1149-1152, 1998, or the like). In this case, a substance that improves the thermostability of an enzyme, such as trehalose also can be added to the reaction solution. This makes it possible to further efficiently amplify a target nucleic acid containing a non-natural nucleotide. Among these DNA polymerases, for example, the polymerase derived from genus *Alicyclohacillus* or genus *Thermus* is preferred, the polymerase derived from *Alicyclohacillus acidocaldarius* or the Taq DNA polymerase is more preferred, and the polymerase derived from genus *Alicyclobacillus* from which the Aac MutS is derived is particularly preferred. Specifically, the polymerase derived from *Alicyclobacillus acidocaldarius* or *Alicyclobacillus acidocaldarius* subsp. *Acidocaldarius* JCM5260 is preferred.

In the first determination method of the present invention, when nucleic acid amplification is conducted by an isothermal amplification method described below, one having a strand displacement activity (strand displacement ability) is preferred as the polymerase, and a normal-temperature, mesophilic, or thermostable polymerase can be used suitably as the same. As the polymerase, one substantially having no 5' →3' exonuclease activity is yet more preferred. Examples of such a polymerase include a Klenow fragment of DNA polymerase I derived from *Escherichia coli* and a variant of the above-mentioned polymerase derived from thermophilic genus *Bacillus,* in which the 5' → 3' exonuclease activity has been deleted. Specific examples of the latter include variants of Bst DNA polymerase and Bca DNA polymerase, in each of which the 5' → 3' exonuclease activity has been deleted.

In the case where the reverse transcription reaction is conducted in the first determination method of the present invention as mentioned above, the enzyme to be used for the reaction is not particularly limited, as long as it has a cDNA synthesizing activity utilizing RNA as a template. Specific examples of the enzyme include an avian myeloblastosis virus derived reverse transcriptase (AMV RTase), a Rous-associated virus-2 reverse transcriptase (RAV-2 RTase), and a Moloney murine leukemia virus derived reverse transcriptase (MMLV RTase). In addition, a DNA polymerase that additionally has reverse transcription activity also can be used for the reverse transcription reaction. Specific examples of the DNA polymerase include a polymerase derived from genus *Thermus* such as a Tth DNA polymerase and a polymerase derived from thermophilic genus Bacillus. Examples of the polymerase derived from thermophilic genus Bacillus include a Bst DNA polymerase, a Bca DNA polymerase, a BcaBEST DNA polymerase, and a Bca (exo-) DNA polymerase. For example, the Bca DNA polymerase does not require manganese ions in a reaction. The Bca DNA polymerase allows cDNA to be synthesized while suppressing the formation of a secondary structure of a template RNA under a high temperature condition.

When, for example, the BcaBEST DNA polymerase, or the Bca (exo-) DNA polymerase is used as the polymerase that also has reverse transcription activity, the reverse transcription reaction using total RNA or mRNA as a template and the DNA polymerase reaction using cDNA obtained by the reverse transcription reaction as a template can be conducted using one type of polymerase. Note here that the enzyme is not limited to this, and the above-mentioned various DNA polymerases and the above-mentioned reverse transcriptase such as MMLV RTase may be used together in combination.

When the MutS is used for the amplification reaction, it is preferred that MutS and the polymerase that are derived from the same are used regardless of the Aac MutS. Specifically, for example, the MutS and the polymerase are derived from the same genus, preferably from the same species, and more preferably from the same strain.

In the first determination method of the present invention, the amount of the enzyme (for example, a polymerase) in the reaction solution is not particularly limited, and is, for example, in the range from 0.01 to 1000 U, preferably from 0.05 to 500 U, and more preferably from 0.1 to 100 U, per 25 µL of the reaction solution.

According to the first determination method of the present invention, the presence or absence of an intron sequence in a sample nucleic acid also can be determined using the intron sequence contained in a genome of a eukaryote as a target site associated with the deletion, insertion, or addition. When the presence or absence of the intron sequence is determined, and it is determined as being absent, it can be determined that mRNA of the target gene is present, i.e., the target gene is expressed. In this case, the target sequence is preferably mRNA.

In the first determination method of the present invention, a nucleic acid amplification method is not particularly limited, and a conventionally known method can be employed. The nucleic acid amplification reaction may be conducted while changing a temperature or at a constant temperature, for example. Examples of the former include a polymerase chain reaction (PCR) method (see, for example, Japanese Patent Nos. 2502041, 2546576, and 2703194) and an RT-PCR method (see, for example, Trends in Biotechnology, Vol. 10, pp. 146-153, 1992). The PCR generally includes: a denaturation step of denaturing a double-stranded nucleic acid into single-stranded nucleic acids; an annealing step of hybridizing a primer to the single-stranded nucleic acid; and an extension step of conducting extension caused by the hybridized primer. The latter is called an isothermal amplification method, and the constant temperature encompasses, for example, not only a temperature that is precisely maintained at a set temperature but also the condition where the temperature is substantially the constant temperature. The "substantially constant temperature" includes the meaning of, for example, the change in temperature to the extent that functions of the various components to be used for an amplification reaction are not impaired. Examples of the isothermal amplification method include a Smart Amplification Process method (see WO 01/030993, WO 2004/040019, WO 2005/063977, and Mitani, Y. et al., Nature Methods, 2007, Vol. 4, No. 3, 257-262), an SDA (strand displacement amplification) method (see JP H10-313900 A), an improved SDA method, an NASBA (nucleic acid sequence based amplification) method (see Japanese Patent No. 2650159), a LAMP (Loop-Mediated Isothermal Amplification) method (see Notomi, T. et al., Nucleic Acids Research, 2000, Vol. 28, No. 12, e63), an ICAN (registered trademark, Isothermal and Chimeric primer-initiated Amplification of Nucleic acids) method (see WO 00/56877), a self-sustained sequence replication (3SR) method, a TMA (transcription-mediated amplification) method, a Q-beta replicase method, an Invader method, and an RCA (rolling circle amplification) method.

The Smart Amplification Process method and the LAMP method, each of which is the isothermal amplification method, and the PCR method are described below as specific examples. Note here that the present invention is not at all limited by these methods.

### (Isothermal amplification method)

The isothermal amplification method generally is a method of conducting a nucleic acid amplification reaction isothermally (at a constant temperature). In the present invention, the conditions of the amplification reaction are not particularly limited and can be decided as appropriate by those skilled in the art. Preferably, the reaction temperature is set at, for example, around the melting temperature (Tm) of the primer or lower. Moreover, the stringency level is set in view of the melting temperature (Tm) of the primer. As specific examples, the reaction temperature is, for example, in the range from about 20°C to about 75°C, and preferably from about 35°C to about 65°C.

The isothermal amplification method is described with reference to a method using an asymmetric primer set and a method using a symmetric primer set as examples. The asymmetric primer set is a primer pair set in which one primer and the other primer are different from each other in morphology and is hereinafter referred to as an "asymmetric primer set". The symmetric primer set is a primer pair set in which one primer and the other primer are identical to each other in morphology and is hereinafter referred to as a "symmetric primer set". The asymmetric primer set is suitable for, for example, the Smart Amplification Process method. The symmetric primer set is suitable for, for example, the LAMP method. Note here that the present invention is not limited by this.

### Smart Amplification Process method

Among the amplification methods, the Smart Amplification Process method can amplify a target sequence with high specificity, for example. Accordingly, the Smart Amplification Process method makes it possible to determine the presence or absence of a mutation in a gene, i.e., the presence or absence of deletion, displacement, insertion, or addition of a base in a gene by a nucleic acid amplification. Specifically, it is suitable for the determination of the presence or absence of a single base mutation (single nucleotide polymorphism).

As mentioned above, the asymmetric primer set is a primer pair set in which one primer and the other primer are different from each other in morphology. Specifically, it is preferred that the asymmetric primer set is applied to the Smart Amplification Process method. Hereinafter, this primer set is also referred to as a "primer set for Smart Amplification Process".

The specific example of the primer set for Smart Amplification Process can be, for example, a pair of primers containing a first primer and a second primer that are asymmetric to each other. The first primer contains, in a 3'-end portion thereof, a sequence (Ac') that hybridizes to a sequence (A) contained in a 3'-end portion of the target sequence. The sequence (Ac') contains, on the 5' side thereof, a sequence (B') that hybridizes to a complementary sequence (Bc) to the sequence (B) that is present on the 5' side with respect to the sequence (A) in the target sequence. The second primer contains, in a 3'-end portion thereof, a sequence (Cc') that hybridizes to a sequence (C) contained in a 3'-end portion of a complementary sequence to the target sequence. The sequence (Cc') contains, on a 5' side thereof, a folded sequence (D-Dc') that contains, on the same strand, two nucleic acid sequences that hybridize to each other thereon.

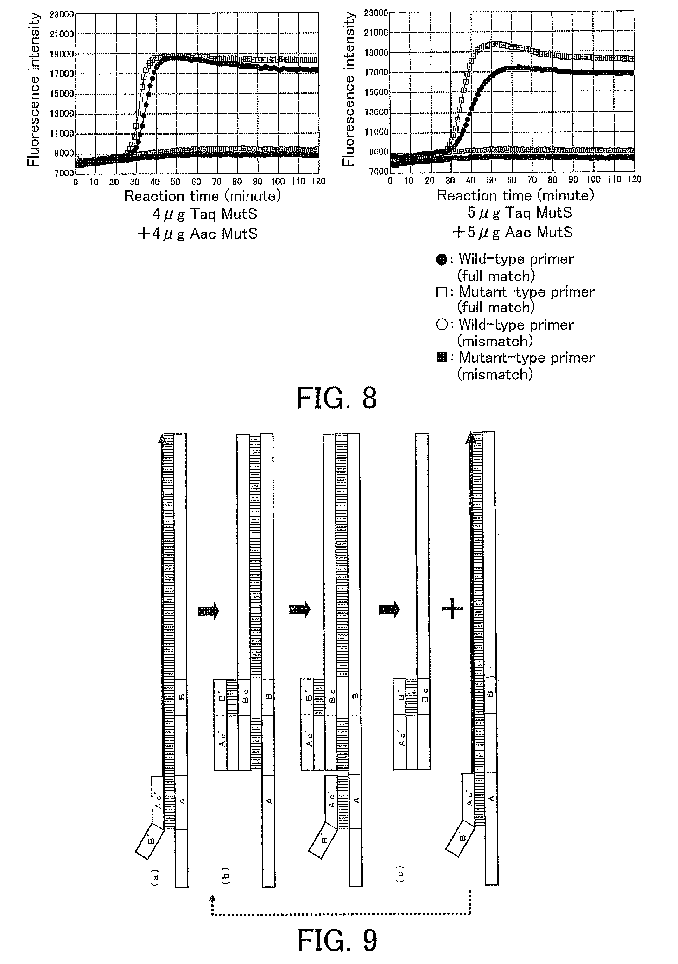

The action mechanism of nucleic acid synthesis using the first primer is schematically shown in FIG. 9. First, a target sequence in a nucleic acid is identified as a template. Then, a sequence (A) in a 3'-end portion of the target sequence and a sequence (B) that is present on a 5' side with respect to the sequence (A) is identified. A first primer contains a sequence (Ac'), and a sequence (B') on the 5' side thereof. The sequence (Ac') hybridizes to the sequence (A), and the sequence (B') hybridizes to a complementary sequence (Bc) to the sequence (B). In this case, the first primer may have, between the sequence (Ac') and the sequence (B'), an intervening sequence that does not affect the reaction. Annealing of such a primer to the template nucleic acid results in a state where the sequence (Ac') of the primer hybridizes to the sequence (A) of the target sequence (FIG. 9, (a)). When a primer extension reaction occurs in this state, a nucleic acid containing the complementary sequence to the target sequence is synthesized. Then, the sequence (B') that is present on the 5' side of the nucleic acid thus synthesized hybridizes to the sequence (Bc) that is present in the nucleic acid. Thus, a stem-loop structure is formed on the 5' side of the synthesized nucleic acid. As a result, the sequence (A) on the template nucleic acid becomes a single strand, and then another primer having the same sequence as that of the first primer hybridizes thereto (FIG. 9, (b)). Thereafter, an extension reaction from a newly hybridized first primer occurs by a strand displacement reaction. At the same time, the nucleic acid that is synthesized previously is dissociated from the template nucleic acid (FIG. 9, (c)).

In the above-described action mechanism, the phenomenon in which the sequence (B') hybridizes to the sequence (Bc) typically occurs because of the presence of complementary regions on the same strand. Generally, when a double-stranded nucleic acid is dissociated into single strands, a partial dissociation starts from ends thereof or the relatively unstable portions other than the ends. In the double-stranded nucleic acid generated through the extension reaction caused by the first primer, base pairs in the end portions are in a state of equilibrium between dissociation and binding at relatively high temperature, whereby a double strand is maintained as a whole. In such a state, when a sequence complementary to the dissociated portion at the end is present on the same strand, a stem-loop structure can be formed in a metastable state. This stem-loop structure does not present stably. However, another identical primer binds to the complementary strand portion (the sequence (A) on the template nucleic acid) exposed because of the formation of the stem-loop structure, whereby polymerase causes the extension reaction to occur immediately. Accordingly, displacement of the strand synthesized previously occurs, and thus it is released. At the same time, a new double-stranded nucleic acid can be generated.

The design criteria for the first primer according to a preferred embodiment of the present invention are as follows. First, in order for a new primer to anneal efficiently to a template nucleic acid after a complementary strand to the template nucleic acid is synthesized through extension of a primer, it is necessary to allow the sequence (A) portion on the template nucleic acid to be a single strand through the formation of a stem-loop structure on the 5' side of the complementary strand that has been synthesized. For that purpose, a ratio of (X-Y)/X is important. That is a ratio of the difference (X-Y) to the X, wherein X indicates the number of bases in the sequence (Ac'), and Y indicates the number of bases in a region between the sequence (A) and the sequence (B) in the target sequence. However, the portion that is present on the 5' side with respect to the sequence (A) on the template nucleic acid and is not associated with the hybridization of the primer is not required to be a single strand. Further, in order for the new primer to anneal efficiently to the template nucleic acid, it is also necessary to form the stem-loop structure efficiently. In order to efficiently form the stem-loop structure, i.e., in order for the sequence (B') to hybridize efficiently to the sequence (Bc), the distance (X+Y) between the sequence (B') and the sequence (Bc) is important. Generally, the optimal temperature for the primer extension reaction is a maximum of around 72°C. It is difficult to dissociate the extended strand over a long region at such low temperature. Thus, conceivably, in order for the sequence (B') to hybridize efficiently to the sequence (Bc), it is preferred that the number of bases between both the sequences is small. In contrast, conceivably, in order for the sequence (B') to hybridize to the sequence (Bc) to allow the sequence (A) portion on the template nucleic acid to be a single strand, it is preferred that the number of bases in the sequence (B') is large.

From the above-described viewpoints, the first primer according to a preferred embodiment of the present invention is designed so that (X-Y)/X is, for example, -1.00 or more, preferably 0.00 or more, more preferably 0.05 or more, and yet more preferably 0.10 or more, or is, for example, 1.00 or less, preferably 0.75 or less, more preferably 0.50 or less, and yet more preferably 0.25 or less, in the case where no intervening sequence is present between the sequence (Ac) and the sequence (B') in the primer. Moreover, (X + Y) is preferably 15 or more, more preferably 20 or more, yet more preferably 30 or more, or is preferably 50 or less, more preferably 48 or less, and yet more preferably 42 or less.

When an intervening sequence (Y' indicates the number of bases therein) is present between the sequence (Ac') and the sequence (B') in the primer, the first primer according to the preferred embodiment of the present invention is designed so that {X-(Y-Y)}/X is, for example, -1.00 or more, preferably 0.00 or more, more preferably 0.05 or more, and yet more preferably 0.10 or more, or is, for example, 1.00 or less, preferably 0.75 or less, more preferably 0.50 or less, and yet more preferably 0.25 or less. Moreover, (X+Y+Y') is preferably 15 or more, more preferably 20 or more, and yet more preferably 30 or more, or is, preferably 100 or less, more preferably 75 or less, and yet more preferably 50 or less.

The first primer has a strand length that enables base pairing with the target nucleic acid while maintaining the necessary specificity under the predetermined conditions, for example. The strand length of this primer is preferably in the range from 15 to 100 nucleotides and more preferably from 20 to 60 nucleotides. The lengths of the sequence (Ac) and the sequence (B') in the first primer are each preferably in the range from 5 to 50 nucleotides and preferably from 7 to 30 nucleotides. An intervening sequence that does not affect the reaction may be inserted between the sequence (Ac) and the sequence (B'), if necessary.

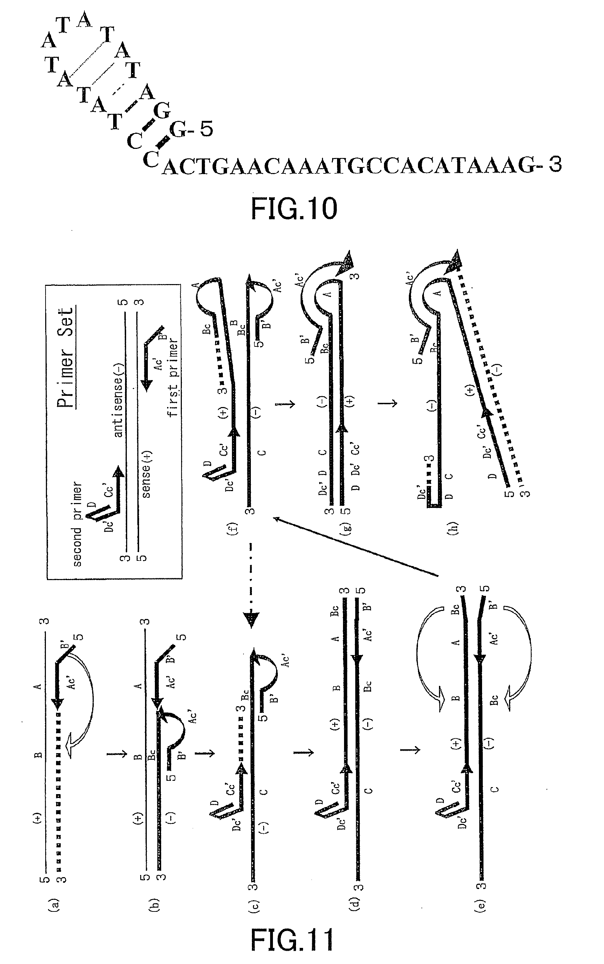

As mentioned above, the second primer contained in the primer set of the present invention contains, in the 3' end portion thereof, the sequence (Cc') that hybridizes to the sequence (C) contained in the 3' end portion of a complementary sequence (the strand that is on the opposite side to the strand to which the first primer hybridizes) to the target sequence. The second primer further contains, on the 5' side of the sequence (Cc'), a folded sequence (D-Dc') that contains, on the same strand, two nucleic acid sequences that hybridize to each other. Such a second primer has a structure shown in FIG. 10, for example. However, the sequence and the number of nucleotides of the second primer are not limited to those shown in FIG. 10. The length of the sequence (Cc') in the second primer is preferably in the range from 5 to 50 nucleotides and more preferably from 10 to 30 nucleotides. The length of the folded sequence (D-Dc') is preferably in the range from 2 to 1000 nucleotides, and more preferably from 2 to 100 nucleotides, yet more preferably from 4 to 60 nucleotides, and even more preferably from 6 to 40 nucleotides. The number of nucleotides of the base pairs that are formed through hybridization in the folded sequence (D-Dc') is preferably in the range from 2 to 500 bp, more preferably from 2 to 50 bp, yet more preferably from 2 to 30 bp, and even more preferably from 3 to 20 bp. The nucleotide sequence of the folded sequence (D-Dc') may be any sequence and is not particularly limited. However, it is preferred that the nucleotide sequence is one that does not hybridize to the target sequence. In addition, an intervening sequence that does not affect the reaction may be inserted between the sequence (Cc') and the folded sequence (D-Dc'), if necessary.

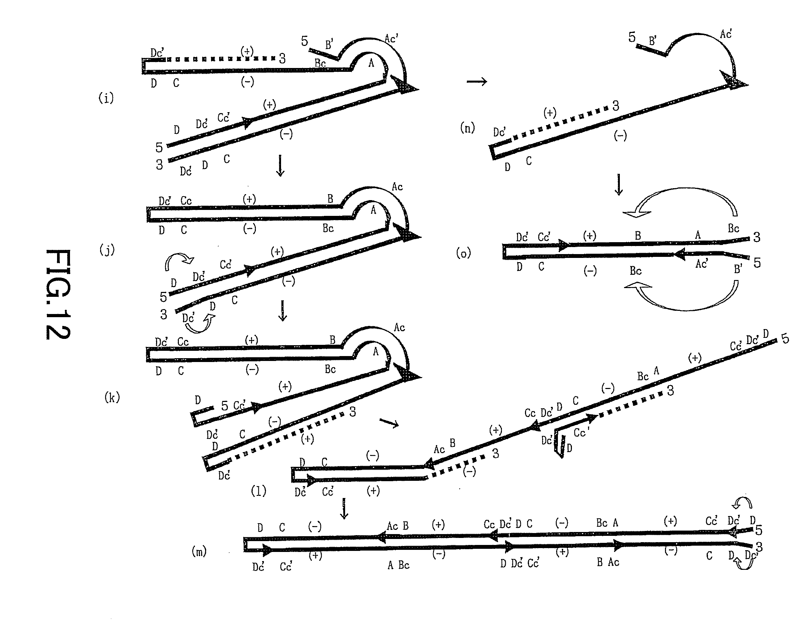

The conceivable action mechanism of the nucleic acid amplification reaction that is caused by the first primer and the second primer is described with reference to FIGs. 11 and 12. In FIGs. 11 and 12, in order to simplify the description, two sequences that hybridize to each other are described as sequences that are complementary to each other. However, the present invention is not limited thereby. First, a first primer hybridizes to a sense strand of a target nucleic acid, whereby an extension reaction caused by the first primer occurs (FIG. 11, (a)). Subsequently, a stem-loop structure is formed on an extended strand (-), whereby a sequence (A) on the sense strand is allowed to be a single strand. Then a new first primer hybridizes to the sequence (A) (FIG. 11, (b)). This causes an extension reaction caused by the first primer, and then the extended strand (-) synthesized previously is dissociated. Next, a second primer hybridizes to a sequence (C) on the extended strand (-) that has been dissociated (FIG. 11, (c)). This causes an extension reaction caused by the second primer, whereby an extended strand (+) is synthesized (FIG. 11, (d)). Stem-loop structures are formed at the 3' end of the extended strand (+) thus generated and at the 5' end of the extended strand (-) (FIG. 11, (e)). Then, an extension reaction occurs from the loop tip of the extended strand (+), being the 3' end of the free form, and at the same time, the extended strand (-) is dissociated (FIG. 11, (f)). The extension reaction from the loop tip results in generation of a hairpin-type double-stranded nucleic acid in which the extended strand (-) has bound on the 3' side of the extended strand (+) through the sequence (A) and the sequence (Bc). Then a first primer hybridizes to the sequence (A) and the sequence (Bc) (FIG. 11, (g)), and an extension reaction caused thereby allows an extended strand (-) to be generated (FIG. 11, (h) and FIG. 12, (i)). Furthermore, the folded sequence that is present at the 3' end of the hairpin-type double-stranded nucleic acid provides the 3' end of the free form (FIG. 11, (h)). Then, an extension reaction caused therefrom (FIG. 12, (i)) allows single stranded nucleic acid to be generated (FIG. 12, (j)). The single-stranded nucleic acid contains the folded sequence at each end thereof and contains the extended strand (+) and the extended strand (-) alternately thorough the sequences derived from the first and second primers. In this single-stranded nucleic acid, the folded sequence that is present at the 3' end thereof provides the 3' end (the origin of complementary strand synthesis) of the free form (FIG. 12, (k)). Accordingly, the similar extension reaction is repeated and the strand length becomes double per one extension reaction (FIG. 12, (l) and (m)). In the extended strand (-) synthesized from the first primer that has been dissociated in FIG. 12, (i), the folded sequence that is present at the 3' end thereof provides the 3' end (the origin of complementary strand synthesis) of the free form (FIG. 12, (n)). Accordingly, the extended reaction caused therefrom allows stem-loop structures to be formed at both ends, whereby a single-stranded nucleic acid is generated (FIG. 12, (o)). The single-stranded nucleic acid contains the extended strand (+) and the extended strand (-) alternately through the sequences derived from the primers. Similarly in this single-stranded nucleic acid, the formation of a loop at the 3' end provides the origin of complementary strand synthesis in succession, whereby an extension reaction caused therefrom occurs in succession. In the single-stranded nucleic acid that is extended automatically in such a manner, the sequences derived from the first primer and the second primer are contained between the extended strand (+) and the extended strand (-). Therefore, each primer can hybridize to cause an extension reaction. This allows the sense strand and the antisense strand of the target nucleic acid to be amplified considerably.

The primer set for Smart Amplification Process further may contain a third primer in addition to the first primer and the second primer. The third primer is, for example, a primer that hybridizes to the target sequence or a complementary sequence thereto and does not compete with other primers for hybridization to the target sequence or the complementary sequence thereto. In the present invention, "does not compete" means that, for example, hybridization of the third primer to a target sequence does not hinder other primers from providing origins of complementary strand synthesis.

When the target sequence is amplified with the first primer and the second primer, the amplification product contains the target sequence and the complementary sequence thereto alternately as mentioned above. The amplification product has, on the 3' side thereof, a folded sequence or a loop structure. It provides the origin of complementary strand synthesis, whereby extension reactions occur consecutively therefrom. It is preferred that when such an amplification product becomes a single strand partially, the third primer can anneal to the target sequence that is present in the single strand portion. This allows the target sequence contained in the amplification product to be provided with a new origin of complementary strand synthesis. Then, an extension reaction occurs therefrom. Thus, the nucleic acid amplification reaction is performed much quicker.

The third primer is not limited, and may be of one type, or for example, in order to improve the speed and specificity of the amplification reaction, two or more types of third primers may be used simultaneously. Typically, such third primers have, for example, different sequences from those of the first primer and the second primer. However, each of the third primers may hybridize to a region, a part of which is hybridized by the first or second primer, as long as they do not compete with the first or second primer. The strand length of the third primer is preferably in the range from 2 to 100 nucleotides, more preferably from 5 to 50 nucleotides, and yet more preferably from 7 to 30 nucleotides.

The third primer is intended mainly to provide an auxiliary function to advance the amplification reaction caused by the first primer and the second primer much quicker. Therefore, it is preferred that the third primer has a lower Tm than that of each 3' end of the first primer and the second primer. Furthermore, it is preferred that the amount of the third primer to be added to the amplification reaction solution is smaller than that of each of the first primer and the second primer to be added thereto, for example.

The third primer can be one that allows an origin of complementary strand synthesis to be provided for a loop portion, with a template having a structure capable of forming the loop, as described in WO 02/24902. The third primer, however, is not limited thereto. That is, it can be any primer that provides an origin of complementary strand synthesis for any site as long as the site is within the target sequence, for example.

In the primer set for Smart Amplification Process, any one or both of the first primer and the second primer may be a labeled primer labeled with, for example, a labeling substance such as a fluorescent dye or the like, or the third primer may be, for example, the labeled primer. Any of one or both of the first primer and the second primer and the third primer may be the labeled primer.

When the Smart Amplification Process method is applied to, for example, a method for determining a mutation, it is preferred that the primer set for Smart Amplification Process is designed as follows. That is, preferably, the primer set for Smart Amplification Process is designed so that, using a nucleic acid sequence (mutant-type sequence) having a mutation at a target site (detection site) thereof or a nucleic acid sequence (wild-type sequence) having no mutation at the target site thereof as a target sequence, the target site at which the target mutation occurs is contained in the sequence (A), (B), or (C) or is located between the sequences (A) and (B) or between the sequences (A) and (C).

When a primer set designed using a mutant-type sequence having a mutation at the target site thereof is used as a target sequence, for example, the presence of an amplification product after the amplification reaction indicates the presence of a mutant-type sequence, while the absence of or reduction in the amplification product after the same indicates the absence of the mutant-type sequence. In contrast, when a primer set is designed using a nucleic acid sequence (wild-type sequence) containing no mutation at the target site thereof is used as a target sequence, for example, the presence of an amplification product after the amplification reaction indicates the absence of the mutant-type sequence, while the absence of or reduction in the amplification product after the same indicates the presence of the mutant-type sequence. The "reduction in the amplification product" means a reduction in the amount of the amplification product to be obtained as compared to the amount of the amplification product that is obtained when the target sequence is present in the sample nucleic acid.

It is preferred that the primer set is designed so that the target site is contained in the sequence (A), for example. With such a primer set, for example, when the target sequence (for example, a wild-type sequence) is contained in the sample nucleic acid, the first primer anneals to the sequence (A) in the amplification reaction. Accordingly, an amplification product is obtained. On the other hand, when a nucleic acid sequence (for example, a mutant-type sequence) that is different from the target sequence is contained in the sample nucleic acid, it is difficult for the first primer to anneal to the sequence (A) in the amplification reaction. Accordingly, no amplification product is obtained or a considerably reduced amount of amplification product is obtained. It is preferred that the sequence (Ac) contained in the first primer is a sequence that is complementary to the sequence (A).

It is preferred that the primer set is designed so that the target site is contained in the sequence (C), for example. With such a primer set, for example, when the target sequence (for example, a wild-type sequence) is contained in a sample nucleic acid, the second primer anneals to the sequence (C) in the amplification reaction. Accordingly, an amplification product is obtained. On the other hand, when a nucleic acid sequence (for example, a mutant-type sequence) that is different from the target sequence is contained in the sample nucleic acid, it is difficult for the second primer to anneal to the sequence (C) in the amplification reaction. Accordingly, no amplification product is obtained or a considerably reduced amount of amplification product is obtained. It is preferred that the sequence (Cc') contained in the second primer is a sequence that is complementary to the sequence (C).

It is preferred that the primer set is designed so that the target site is contained in the sequence (B), for example. With such a primer set, for example, when the target sequence (for example, a wild-type sequence) is contained in a sample nucleic acid, the first primer anneals to the sequence (A) to cause an extension reaction, and thereafter the sequence (B') contained in the primer hybridizes to the sequence (Bc) located on the extended strand in the amplification reaction. Therefore, a stem-loop structure is formed efficiently. This efficient formation of the stem-loop structure allows another first primer to anneal to the template. Accordingly, the action mechanism shown in FIG. 9 proceeds efficiently, whereby an amplification product is obtained. On the other hand, when a nucleic acid sequence (for example, a mutant-type sequence) that is different from the target sequence is contained in the sample nucleic acid, it is difficult to form the stem-loop structure in the amplification reaction. Accordingly, the action mechanism shown in FIG. 9 is hindered. As a result, no amplification product is obtained or a considerably reduced amount of amplification product is obtained. It is preferred that the sequence (B') contained in the first primer is a sequence that is identical to the sequence (B).

It is preferred that the primer set is designed so that the target site is located between the sequence (A) and the sequence (B), for example. With such a primer set, when the target sequence (for example, a wild-type sequence) is contained in a sample nucleic acid, the first primer anneals to the sequence (A) to cause an extension reaction, and thereafter, the sequence (B') contained in the primer hybridizes to the sequence (Bc) located on the extended strand in an amplification reaction. Therefore, a stem-loop structure is formed efficiently. This efficient formation of the stem-loop structure allows another first primer to anneal to the template. Accordingly the action mechanism shown in FIG. 9 proceeds efficiently, whereby an amplification product is obtained. On the other hand, when a nucleic acid sequence (for example, a mutant-type sequence) that is different from the target sequence is contained in the sample nucleic acid, it is difficult to form the stem-loop structure in the amplification reaction because the distance maintained between the sequence (B') contained in the first primer and the sequence (Bc) located on the extended strand is not appropriate. This is, for example, the case where there is insertion or deletion of a long sequence between the sequence (A) and the sequence (B). Thus, in this case, the action mechanism shown in FIG. 9 is hindered. As a result, no amplification product is obtained or a considerably reduced amount of amplification product is obtained.

It is preferred that the primer set is designed so that the target site is located between the sequence (A) and the sequence (C), for example. With such a primer set, when the target sequence (for example, a wild-type sequence) is contained in a sample nucleic acid, the first primer anneals to the sequence (A) to cause an extension reaction, and thereafter, the sequence (B') contained in the primer hybridizes to the sequence (Bc) located on the extended strand in an amplification reaction. Therefore, a stem-loop structure is formed efficiently. This efficient formation of the stem-loop structure allows another first primer to anneal to the template. Accordingly, the action mechanisms shown in FIGs. 9, 11, and 12 proceed efficiently. Thus, an amplification product is obtained. On the other hand, when a nucleic acid sequence (for example, a mutant-type sequence) that is different from the target sequence is contained in the sample nucleic acid, no amplification product is obtained or a considerably reduced amount of amplification product is obtained. For example, when the sample nucleic acid contains a nucleic acid sequence that is different from the target sequence because of the insertion of a long sequence between the sequences (A) and (C), the rate (efficiency) of amplification is reduced considerably. As a result, no amplification product is obtained or a considerably reduced amount of amplification product is obtained. Furthermore, when the sample nucleic acid contains a nucleic acid sequence that is different from the target sequence because of the deletion of a sequence between the sequence (A) and the sequence (C), and a part or the whole of the sequence (B) has been lost because of the deletion, the sequence (B') contained in the first primer cannot hybridize onto the extended strand. Accordingly, a stem-loop structure cannot be formed or forms with difficulty. Thus, the action mechanisms shown in FIGs. 9, 11, and 12 are hindered. As a result, no amplification product is obtained or a considerably reduced amount of amplification product is obtained. Moreover, also when the sample nucleic acid contains a nucleic acid sequence that is different from the target sequence because of the deletion of a sequence between the sequence (A) and the sequence (C), and no partial deletion of the sequence (B) is caused by the deletion, the rate (efficiency) of amplification is reduced. As a result, no amplification product is obtained or a considerably reduced amount of amplification product is obtained.

In the present invention, the target site associated with the above-mentioned deletion, insertion, or addition may be an intron sequence contained in a genome of a eukaryote. In this case, it is preferred that a primer set is used that is designed so that a target site associated with the deletion of an intron sequence is located between the sequences (A) and (B) using mRNA with an intron having been deleted of the target gene as a sample nucleic acid. With such a primer set, first, the sequence (Ac') that is present on the 3' side of the first primer anneals to the template nucleic acid (sample nucleic acid) to cause an extension reaction. Then, only when a target region is synthesized by the extended strand produced by the first primer, the sequence (B') that is present on the 5' side of the first primer can hybridize to the sequence (Bc) corresponding to exon that is next thereto on a self-extension strand. That is, it is not until the target region of mRNA having a sequence with two exons joined sequentially is synthesized by the extended strand that a stem-loop structure shown in FIG. 9 is formed, which allows a new first primer to anneal to the sequence (A) in the template nucleic acid that has been a single strand. As mentioned above, the formation of the stem-loop structure on the 5' side of this first primer is repeated efficiently when the sequences (A) and (B) in the template nucleic acid are present at a suitable interval. Accordingly, only when mRNA containing no intron sequence is used as a template, amplification occurs, while no amplification occurs in genomic DNA containing an intron sequence. When this reaction is repeated isothermally, the target sequence can be amplified accurately, and the formation of the stem-loop structure is repeated accurately for every cycle. Thus only the target sequence can be amplified accurately. Specifically, specificity is high in such a Smart Amplification Process method. Thus, nonspecific amplification is suppressed, and only a target mRNA can be specifically amplified, whereby quantitative ability is improved. According to the present invention, the Aac MutS of the present invention is caused to be present, whereby the quantitative ability can be further improved. Furthermore, this principle makes it possible to abbreviate a step of obtaining RNA by a complicated, time-consuming DNase treatment to break DNA contained in a sample. Thus, spontaneous decay of mRNA can be reduced, whereby quicker qualitative or quantitative diagnosis can be conducted.

### LAMP method

As mentioned above, the symmetric primer set is a primer pair set in which one primer and the other primer are identical to each other in morphology. Specifically, it is preferred that the symmetric primer set is applied to the LAMP method. Hereinafter, this primer set is also referred to as a "primer set for LAMP".

In the LAMP method, for example, four types of primers are necessary. They recognize six regions, so that a target gene can be amplified. That is, in this method, first, a first primer anneals to a template strand to cause an extension reaction. Subsequently, the extended strand produced by the first primer separates from the template strand because of the strand displacement reaction caused by a second primer designed upstream from the first primer. At the time, a stem-loop structure is formed on the 5' side of the extended strand because of the structure of the extended strand of the first primer, which has been removed. Similar reactions occur in the other strand of the double-stranded nucleic acid or on the 3' side of the extended strand of the first primer, which has been removed. These reactions are repeated, whereby the target sequence is amplified. The template used in the LAMP method has, for example, at the 3' side and the 5' side on the same strand, regions having base sequences complementary to each other in the respective end regions. With this template (also referred to as "dumbbell-type template nucleic acid"), loops are formed, in which base pairing can occur between the base sequences that are complementary to each other when they anneal to each other. The LAMP method can be performed according to, for example, WO 00/28082 or WO 01/034838.

### (Non-isothermal amplification method)

### PCR method

As mentioned above, in the PCR method, a target sequence can be amplified through dissociation of a double-stranded nucleic acid, annealing of a primer to a single strand obtained by the dissociation, and a nucleic acid synthesis caused by the primer with a change in reaction temperature. The conditions of the PCR method are not particularly limited, and can be set as appropriate by those skilled in the art.

The first determination method of the present invention is described below with reference to an example using a double-stranded DNA as a sample nucleic acid (template nucleic acid).

First, a reaction solution containing a double-stranded DNA as a sample nucleic acid, a primer, Aac MutS, a DNA polymerase, and dNTP is prepared. The type of the primer to be used is not particularly limited, and can be set according to, for example, types of a nucleic acid amplification reaction and a target sequence to be amplified. One or more types of primers or one or more types of primer pair sets may be used.

The concentrations of the respective components in the reaction solution are not particularly limited, and are, for example, the same as those mentioned above. The concentration of the dNTP in the reaction solution is, for example, in the range from 0.01 to 100 mmol/L and preferably from 0.1 to 10 mmol/L. For example, the dNTP contains ATP, TTP, GTP, and CTP and may contain UTP as substitute for or in addition to TTP.

The reaction solution further may contain, for example, a buffer solution, a surfactant, a catalyst, DMSO (dimethyl sulfoxide), betaine, DTT (dithiothreitol), a chelating reagent such as EDTA, or glycerol. Examples of the buffer solution include a tris-HCL buffer solution, a tricine buffer solution, a sodium phosphate buffer solution, and a potassium phosphate buffer solution. The concentration of the buffer solution in the reaction solution is, for example, in the range from 0.001 to 1000 mmol/L, and the pH of the same is, for example, in the range from 5 to 10. Examples of the surfactant include Tween such as Tween-20 and Triton such as Triton X-100. Examples of the catalyst include a potassium salt such as potassium acetate, an ammonium salt such as ammonium sulfate, and a magnesium salt such as magnesium sulfate. For example, the reaction solution further may contain DMSO, betaine, formamide, glycerol, or the like as a melting temperature regulator for improving nucleic acid amplification efficiency. For example, the reaction solution further may contain glycerol, bovine serum albumin, a sugar, or the like as an enzyme stabilizer for stabilizing an enzyme. Examples of the sugar include monosaccharide and oligosaccharide, and specifically, trehalose, sorbitol, mannitol, or the like can be used. The reaction solution further may contain any of acid substances and cation complexes described in WO 99/54455 and the like. These components may be used alone or in a combination of two or more of them, for example.

Then, as mentioned above, a nucleic acid amplification reaction is conducted with the Aac MutS present in the reaction solution. The conditions of the amplification reaction are not particularly limited and can be set as appropriate according to the type thereof.

Further, an amplification product obtained by the amplification reaction is detected, and then the presence or absence of amplification is checked. The detection of the amplification product may be conducted over time during the reaction or may be conducted after the elapse of a certain period of time from the start of the reaction. The former is a detection in real time and may be a continuous detection or a intermittent detection. In the latter case, for example, it is preferred that the detection of an amplification product is conducted at the start of the reaction and after the elapse of a certain period of time, and the presence or absence of amplification is checked on the basis of the change in detection result.

The method for detecting an amplification product is not particularly limited, and conventionally known methods described below can be used.

The method for detecting an amplification product can be, for example, a method for detecting an amplification product with a specific size by general gel electrophoresis. The amplification product can be detected using a fluorescent substance such as ethidium bromide or SYBR (registered trademark) Green. It can be detected also by allowing a probe labeled with a labeling substance to hybridize to the amplification product. The labeling substance can be, for example, biotin. The biotin can be detected by binding it to, for example, fluorescently-labeled avidin or avidin that has bound to an enzyme such as peroxidase. Further, it can be detected by a method using an immunochromatograph, and the method can be, for example, a method (the immunochromatography method) using a chromatograph medium in which a macroscopically detectable label is used. Specifically, the amplification product and a labeled probe are hybridized to each other, and an amplification product thus obtained then is contacted with the chromatographic medium on which a capturing probe that can hybridize to the amplification product at a site that is different from the labeled probe has been immobilized. Accordingly, a hybrid body between the amplification product and the labeled probe can be trapped by the capturing probe that has been immobilized on the chromatographic medium. As a result, it becomes possible easily to detect the amplification product by the naked eye. In the present invention, for example, it is also possible to detect indirectly an amplification product thorough the detection of pyrophoric acid that is a byproduct of amplification. Specifically, amplification efficiency is high in the Smart Amplification Process method. Thus, the indirect detection through the detection of pyrophoric acid also is preferred. In such a method, for example, the presence or absence of amplification can be detected by observing cloudiness of the reaction solution by the naked eye or the optical manner, utilizing the fact that generated pyrophoric acid binds to magnesium in the reaction solution, and thus a white precipitate of magnesium pyrophosphate is generated. Moreover, it can be detected by a method utilizing the fact that the magnesium ion concentration is reduced considerably when an insoluble salt is formed by binding pyrophoric acid strongly to metal ions such as magnesium ions. In such a method, when a metal indicator (for example, Eriochrome Black T or Hydroxy Naphthol Blue) whose color tone changes according to the magnesium ion concentration is added previously to the reaction solution, the presence or absence of amplification can be detected by observation through a visual check or the optical manner. Further, in this method, when a fluorescent dye such as Calcein is used, for example, the increase in fluorescence according to an amplification reaction can be observed by visual check or the optical method. This allows the amplification product to be detected in real time.

In the present invention, the presence or absence of an amplification product can be detected also by observing the aggregation of a solid-phase support that results from the generation of the amplification product. In such a method, it is preferred that, for example, at least one type of primer used for the present invention has bound to a solid-phase support or has a site or a group that can bind to the solid-phase support. In the primer, the solid-phase support or the site or the group that can bind to the solid-phase support may be, for example, introduced into any region such as the 3' end region, the 5' end region, or the center region of the primer and is preferably introduced into the 5' end region of the primer. Furthermore, a substrate to be used for the amplification reaction, such as deoxynucleotide (dNTP) may bind to a solid-phase support or may contain the site or the group that can bind to the solid-phase support, for example.

The solid-phase support is not particularly limited, and for example, a support that is insoluble in the reaction solution to be used for the amplification reaction or a phase transition support whose state changes from a liquid phase to a solid phase (gel phase) or from a solid phase (gel phase) to a liquid phase before and after the amplification can be used. Examples of preferred solid-phase support include a water-insoluble organic polymer support, a water-insoluble inorganic polymer support, a synthetic polymer support, a phase transition support, a metal colloid, a magnetic particle, a solvent-insoluble organic polymer support, a solvent-insoluble inorganic polymer support, a solvent-soluble polymer support, and a gel polymer support. Examples of the water-insoluble organic polymer include: silicon-containing substances such as porous silica, porous glass, diatomaceous earth, and celite; cross-linked polysaccharide such as nitrocellulose, hydroxyapatite, agarose, dextran, cellulose, and carboxymethyl cellulose; cross-linked protein such as methylated albumin, gelatin, collagen, and casein; gel-like particles; and dye sol. Examples of the water-insoluble inorganic polymer include aluminum oxide, titanium oxide, and ceramic particles. Examples of the synthetic polymer include polystyrene, poly(meth)acrylate, polyvinyl alcohol, polyacrylonitrile, copolymers thereof, a styrene-styrenesulfonic acid copolymer, and a vinyl acetate-acrylic ester copolymer. The metal colloid can be, for example, a gold colloid. Examples of the magnetic particle include magnetic iron oxide beads, support particles whose surfaces have been coated with pulverized particles of magnetic iron oxide, superparamagnetic particles (JP H4-501959 A), magnetically responsive particles having superparamagnetic iron oxide that is covered with a coating film of polymeric silane (JP H7-6986 B), and magnetizable particles of a fine powder enclosed in an organic polymer. The magnetized solid-phase support easily can separate a solid and a fluid utilizing magnetic force, for example. The form of the solid-phase support is not particularly limited, and examples thereof include particle, film, fiber, and filter. The form is preferably particle among them. The surface of the particle may be any of porous or non-porous. Examples of particularly preferred solid-phase support include: latex in which synthetic polymer supports are dispersed uniformly in water or the like; metal colloidal particles such as gold colloids; and magnetic particles such as magnetic beads.

The method for immobilizing the primer or the substrate on the solid-phase support is not particularly limited. The immobilization can be conducted by a method that is known to those skilled in the art, and the method can use any of physical bonds and chemical bonds. Generally, the immobilization can be conducted using a combination of a substance that can label oligonucleotide such as a primer or a probe and a solid-phase support to which a substance that can bind to the above-mentioned substance has bound, for example. The combination of the substances is not particularly limited, and those well-known in the art can be used. Examples thereof include a combination of biotin and avidin or streptavidin, a combination of an antigen and an antibody that can bind thereto, a combination of ligand and a receptor that can bind thereto, and a combination of two nucleic acids that hybridize to each other. Specifically, for example, a primer or a substrate labeled with biotin is caused to bind to a solid-phase support whose surface has been coated with avidin or streptavidin, so that the primer or the substrate can be immobilized on the solid-phase support. The antigen can be, for example, hapten such as FITC, DIG, or DNP. Examples of the antibody that can bind to them include an anti-FITC antibody, an anti-DIG antibody, and an anti-DNP antibody. These antibodies each may be, for example, any of a monoclonal antibody and a polyclonal antibody. Specifically, a binding between biotin and streptavidin is particularly preferred because of having high specificity and high binding efficiency, for example. A labeling substance such as biotin, hapten, or ligand can be introduced into the 5' end region of a primer alone, or if necessary in a combination of two or more of them, by a well-known method (see, for example, JP S59-93099 A, JP S59-148798 A, and JP S59-204200 A).

The site or the group that can bind to the solid-phase support can be selected as appropriate according to the above-mentioned methods for immobilizing a primer or a substrate on the solid-phase support. Accordingly, the site or the group can be any of the one that can bind physically to the solid-phase support and the one that can binds chemically to the same. However, it is preferred that the site or the group allow a solid-phase support to bind specifically thereto. Examples of the site that can bind to the solid-phase support includes those described above such as biotin, avidin, streptavidin, an antigen, an antibody, a ligand, a receptor, a nucleic acid, and a protein. The site is preferably biotin or streptavidin and more preferably biotin. The use of a primer or a substrate that contains such a site allows the solid-phase support to bind to an amplification product generated after the amplification reaction, for example. In this case, the solid-phase support preferably contains, for example, a binding partner for the site that is contained in the primer or the substrate, if necessary. It is only necessary that the binding partner contained in the solid-phase support is present in the state that it can bind to the site contained in the primer or the substrate. Preferably, the binding partner is present on the surface of the solid-phase support, and more preferably, it is one with which a surface of the solid-phase support has been coated.

In the present invention, for example, the primer set such as mentioned above is provided for each of plural target sequences, these plural primer sets are immobilized on the respective solid-phase supports in such a manner as to be distinguishable from each other, and then the amplification reaction is conducted using these primer sets thus immobilized. With such a method, it is possible to amplify plural target sequences simultaneously and to distinguish among and detect amplification products of the respective target sequences. The amplification products can be detected using, for example, an intercalator. Specifically, for example, the plural primers are each immobilized on the specific position of a planar solid-phase support, so that, after the amplification reaction and the detection of amplification products, the target sequences thus amplified can be specified on the basis of the positions where the amplification products are detected. The solid-phase support to be used for such a method can be not only the planar solid-phase support but also, for example, the one that is well-known in the art, such as surfaces of beads that are distinguishable from one another (the specifications of U.S. Pat. Nos. 6046807 and 6057107) or a sub-planar support that is produced by bundling those obtained by solid-phasing the respective primer sets on fibrous supports, which is then is cut into thin sections (JP 2000-245460 A).

Besides these methods, the method for detecting an amplification product can be, for example, an intercalator method. This is a method in which the presence or absence of amplification is determined on the basis of a fluorescence generated in response to an irradiation of an excitation light using an intercalator that intercalates into a double-stranded nucleic acid. Moreover, a method utilizing a fluorescent substance and quencher also can be employed, and examples thereof include a TaqMan (registered trademark) probe method and a cycling probe method. It is also preferred that the presence or absence of amplification is determined using a probe or a primer, having a compound disclosed in WO 2008/111485. According to this method, when a double-stranded nucleic acid between the probe or the primer and an amplification product is formed, fluorescence is generated in response to an irradiation of an excitation light. Thus, the presence or absence of amplification can be determined on the basis of the detection of the fluorescence. This method is particularly preferred because it allows an increase in background to be reduced even when a nucleic acid sample is not purified or poorly purified. It is preferred that these methods are applied to the detection in real time.

It is also possible that the 5' end of a primer is previously immobilized on a solid phase such as a chip, and then, the amplification reaction is conducted thereon. In this case, a fluorescent substance that emits light by formation of a double strand may be added previously to the primer, or the amplification reaction may be conducted in the presence of the probe to which the fluorescent substance has been added. This allows the amplification product to be detected in real time while conducting the amplification reaction on the solid phase such as the chip.

Then, whether the target site in a sample nucleic acid sequence is of a wild type or a mutant type is determined on the basis of the presence or absence of amplification. In the case where, for example, a primer that is completely complementary to a region including the target site contained in the wild-type sequence is used as the primer, when amplification is found, it can be determined that the target site is of a wild type, whereby a mutation is not present. In the same case, when amplification is not found, it can be determined that the target site is of a mutant type, whereby a mutation is present. On the other hand, in the case where, for example, a primer that is completely complementary to a region including the target site contained in a mutant-type sequence is used as the primer, when amplification is found, it can be determined that the target site is of a mutant type, whereby a mutation is present. In the same case, when amplification is not found, it can be determined that the target site is of a wild type, whereby a mutation is not present.

Next, the second determination method of the present invention is described.

As mentioned above, the second determination method of the present invention is a method for determining the presence or absence of a mutation at a target site contained in a sample nucleic acid. The method includes the steps (I') and (II) below:
(I') the step of amplifying a target sequence including the target site contained in the sample nucleic acid using a primer for amplifying the sample nucleic acid in a presence of the Aac MutS of the present invention and a probe that can hybridize to the region including the target site contained in the sample nucleic acid; and
(II) the step of checking for presence or absence of amplification.

As mentioned above, the Aac MutS used for the determination method of the present invention can specifically recognize and bind to a mismatched base pair and has higher specificity to a mismatched base pair than that to a fully-matched base pair. Thus, according to the second determination method of the present invention, when a probe that can hybridize to a region including the target site contained in the sample nucleic acid binds to the sample nucleic acid with a mismatch, the Aac MutS binds specifically to a site with the mismatch. In this case, even when an extended strand from a primer that has been hybridized to a region that is different from that to which the probe has been hybridized in the sample nucleic acid reaches to the vicinity of the site with the mismatch, an extension reaction is suppressed because of the presence of the Aac MutS that has bound the site with the mismatch. As a result, wrong amplification of the target sequence that binds to the probe with a mismatch can be prevented from occurring. Thus, the presence or absence of a mutation can be determined on the basis of the presence or absence of amplification with high reliability.

The probe is also referred to as a "target probe" because it can hybridize to a region including a target site, and the "region including a target site" is also referred to as a hybrid region as referred in the first determination method because the target probe can hybridize thereto.

The second determination method of the present invention can be conducted in the same manner as the first determination method of the present invention unless otherwise indicated. Specifically, the second determination method can be conducted in the same manner as the first determination method except that the above-described target probe is used as substitute for the "target primer" used for the first determination method of the present invention and a primer for amplifying the target sequence further is used.

In the above-described target probe, the types of nucleic acid and bases and the like can be the same as those in the target primer used in the first determination method. The length of the probe is not particularly limited, and is, for example, in the range from 5 to 40 bases and more preferably from 15 to 25 bases. The conditions under which the probe anneals to the sample nucleic acid are not particularly limited, and it is preferred that, for example, the probe hybridizes to the sample nucleic acid at the temperature in the range from 20°C to 80°C. Moreover, the probe may have a label or an active group such as an amino group at one or both of its ends.

In the second determination method of the present invention, in the case where a target probe that can hybridize to the region in which the target site is of a mutant type is used for the step (I'), when amplification is found in the step (II), it can be determined that the target site is of a mutant type. In the same case, when amplification is not found in the same, it can be determined that the target site is of a normal type. On the other hand, when a target probe that can hybridize to the region in which the target site is of a wild type is used for the step (I'), when amplification is found in the step (II), it can be determined that the target site is of a normal type. In the same case, when amplification is not found in the same, it can be determined that the target site is of a mutant type.

### <Elongation reaction suppression method and nucleic acid amplification method>

The suppression method of the present invention is a method for suppressing an extension reaction caused by a primer that has bound to a sample nucleic acid with a mismatch. In the suppression method, a target sequence contained in the sample nucleic acid is amplified using a primer for amplifying the target sequence contained in the sample nucleic acid in the presence of the Aac MutS of the present invention.

The nucleic acid amplification method of the present invention is a method for amplifying a target sequence contained in a sample nucleic acid. The method includes the step of amplifying the target sequence contained in the sample nucleic acid using a primer for amplifying the target sequence. In the step, an extension reaction caused by the primer that has bound to the sample nucleic acid with a mismatch is suppressed by the suppression method of the present invention.

As mentioned above, the Aac MutS of the present invention binds specifically to a mismatched base pair contained in a double-stranded nucleic acid. Therefore, in the case where the target sequence is amplified in the presence of the Aac MutS of the present invention, when the primer binds to the sample nucleic acid with a mismatch, the Aac MutS recognizes and binds to a mismatched base pair. Thus, the extension reaction caused by the primer can be suppressed. On the other hand, as mentioned below, there is a method for determining the presence or absence of a mutation in a target site on the basis of the presence or absence of amplification using a primer. In this case, when the mismatched base pair is formed between the sample nucleic acid and the primer by the presence of the Aac MutS of the present invention, the Aac MutS recognizes and binds to the mismatched base pair. Thus, extension from the primer is suppressed. Further, the Aac MutS of the present invention has high specificity specifically to the mismatched base pair as mentioned above, whereby the presence or absence of a mutation can be determined with higher reliability than ever before.

There is a case where a site at which it is possible to generate a mutation in the target sequence of the sample nucleic acid is known. For example, when, using the site as a target site, amplification of the target sequence is controlled, or elongation from a primer is suppressed on the basis of whether the target site is of a wild type or a mutant type, the nucleic acid amplification method and suppression method of the present invention preferably includes the step (I) or (I') below. Note here that steps (I) and (I') are the same as those in above-mentioned determination method of the present invention.
(I) the step of amplifying a target sequence including the target site contained in the sample nucleic acid using a primer that can hybridize to a region including the target site contained in the sample nucleic acid in a presence of the Aac MutS of the present invention
(I') the step of amplifying a target sequence including the target site contained in the sample nucleic acid using a primer for amplifying the sample nucleic acid in a presence of the Aac MutS of the present invention and a probe that can hybridize to the region including the target site contained in the sample nucleic acid

The suppression method and the nucleic acid amplification method of the present invention include conducting an amplification reaction in the presence of the Aac MutS of the present invention. Other conditions are not particularly limited. Specific means for these methods are the same as the above-mentioned method for determining a mutation of the present invention.

### <Various regents>

The determination reagent of the present invention is used for the determination method of the present invention. The reagent contains the Aac MutS of the present invention. As long as the determination reagent of the present invention contains the Aac MutS of the present invention, the other components are not at all limited.

The determination reagent of the present invention may further contain the above-mentioned additive such as ADP, other MutS, a primer, an enzyme such as a polymerase, a reagent such as dNTP, a buffer solution, a melting temperature regulator, or a enzyme stabilizing agent. The amount of each component to be added to the determination reagent of the present invention is not particularly limited and is, preferably, the amount with which the concentration thereof becomes the above-mentioned concentration when it is added to the reaction solution of the amplification reaction. Moreover, the determination reagent of the present invention may be, for example, a determination kit used for the determination method of the present invention. In this case, for example, the determination kit preferably includes instructions thereof. In the determination reagent and determination kit of the present invention, the components may be contained in the respective containers individually, or may be combined and-contained in the respective containers, for example. The form or the material of the container is also not particularly limited.

An amplification reagent of the present invention is used for the amplification reaction of the present invention. A suppression reagent of the present invention is used for the suppression method of the present invention. They each contain the Aac MutS of the present invention. As long as the amplification reagent and the suppression reagent of the present invention each contain the Aac MutS of the present invention, the other configurations are not at all limited. The components are not particularly limited and are the same as those of the above-mentioned determination reagent.

Next, the examples of the present invention are described. Note here that, however, the present invention is not limited by the following examples.

### Examples

### [Example 1]

Aac MutS was expressed and purified by cloning DNA from *Alicyclohacillus acidocaldazius* subsp. *Acidocaldarius* JCM5260.

### Expression of Aac MutS

DNA having a base sequence of SEQ ID NO: 1 that encodes Aac MutS was inserted into an NdeI-EcoRI site of a pET17b vector (produced by Novagen) using an In-Fusion PCR cloning kit (produced by Takara Bio Inc.). Thus, an Aac MutS expression vector, pETAacmutS, was constructed. The pETAacmutS was introduced into *Escherichia coli*, BL21-CodonPlus(DE3)RIL (produced by Stratagene), which was then subjected to shaking culture in 100 mL of LB medium containing 50 µg/mL carbenicillin and 34 µg/mL chloramphenicol at 37°C overnight. Thus, a preculture solution was obtained. 5 mL of the preculture solution was inoculated into 500 mL of LB medium containing 100 µg/mL ampicillin and 34 µg/mL chloramphenicol, which was then subjected to shaking culture at 33°C and 200 rpm. At the time when the OD600 of this culture solution reached around 1, IPTG was added to the culture solution so that the final concentration thereof was 0.1 mmol/L, which then was subjected to further shaking culture at 33°C and 200 rpm for 3 hours. Subsequently, this culture solution was transferred into a centrifuge tube, which then was subjected to centrifugation at 39,200 m/s² for 4 minutes. Thus bacterial cells were collected. The bacterial cells thus collected were suspended in 50 mL of PBS, which again was then subjected to centrifugation at 39,200 m/s² for 4 minutes. Thus the bacterial cells were washed. The bacterial cells were suspended in 5 mL of lysis buffer per 1 g of the bacterial cells and then broken by a French press under the condition of 6.2 MPa. The composition of the lysis buffer included a 50 mmol/L tris-HCl buffer solution (pH7.5), 5 mmol/L EDTA, 5 mmol/L 2-mercaptoethanol, 25% (w/v) sucrose, and a protease inhibitor tablet (1 tablet/L, Complete EDTA-free Protease inhibitor cocktail tablets (product name), produced by Hoffmann-La Roche Ltd). 10% Brij-58 was added to this solution containing broken bacterial cells so that the final concentration thereof was 0.5% (w/v), which were then gently mixed together at 4°C for 30 minutes. This mixed solution thus obtained was subjected to centrifugation at 4°C and 15,000 rpm for 40 minutes. Thus, a supernatant was obtained. 30 mL of the supernatant was transferred into 50 ml-capacity tube (produced by Falcon), which then was subjected to a heat treatment at 60°C for 10 minutes. The supernatant after the heat treatment was subjected to centrifugation at 4°C and 18,000 rpm for 40 minutes. Thus, a supernatant was obtained. This supernatant thus obtained was dialyzed with 4 L of a running buffer two times. Thus, a crude extract was obtained. The composition of the running buffer included 50 mmol/L tris-HCl buffer solution (pH7.5), 5 mmol/L EDTA, and 5 mmol/L 2-mercaptoethanol.

### Purification of Aac MutS

Aac MutS was purified using various chromatographies.

### (1) Strong anion exchange column chromatography

A strong anion exchange column (Resource Q (50mL), produced by GE Healthcare) and a high-performance liquid chromatography system (AKTA explorer 100, produced by GE Healthcare) were used. The strong anion exchange column was equilibrated using a first running buffer under the condition where a flow rate was 2 mL/minute. The composition of the first running buffer included 50 mmol/L tris-HCl buffer solution (pH7.5), 5 mmol/L EDTA, 5 mmol/L 2-mercaptoethanol, and 10% (w/v) glycerol. Then, the crude extract was applied to the strong anion exchange column at a flow rate of 3 mL/minute. Thereafter, 120 mL of the first running buffer was passed through the column under the same condition as that of the application, so that the column was washed and non-adsorbed fractions were removed. Subsequently, 540 mL of the first running buffer containing sodium chloride with a concentration gradient from 0 to 300 mmol/L and then, 540 mL of the first running buffer containing sodium chloride with a concentration gradient from 300 to 1000 mmol/L were passed through the column, so that adsorbed fractions were eluted. The adsorbed fractions were fractionated by 10 mL. Each fraction was subjected to SDS-polyacrylamide gel electrophoresis (SDS-PAGE) to check a protein band with the desired molecular weight (MW of about 96,000 Da), and then, the fractions having the protein band were collected. These fractions thus collected were gathered together, which was then subjected to centrifugation at 4°C and 49,000 m/s² for 15 minutes using Amicon Ultra-15 (Produced by Millipore Corporation) so that it was concentrated to about 20 mL. The first running buffer (containing no sodium chloride) with the same amount as that of this concentrated solution was added thereto, which was then again subjected to centrifugation using Amicon Ultra-15 in the same manner as that of the concentration. The first running buffer was added to a concentrated solution thus obtained so that the total amount was 50 mL.

### (2) Heparin affinity column chromatography

Next, a heparin affinity column (heparin sepharose HP (50mL), produced by GE Healthcare) and the high-performance liquid chromatography system were used. The heparin affinity column was equilibrated using the first running buffer under the condition where a flow rate was 2 mL/minute. The solution obtained by the above-mentioned ion exchange column chromatography was applied to the heparin affinity column at a flow rate of 1 mL/minute. Thereafter, 25 mL of the first running buffer was passed through the column under the same condition as that of the application, so that the column was washed and non-adsorbed fractions were removed. Subsequently, 400 mL of the first running buffer containing sodium chloride with a concentration gradient from 0 to 450 mmol/L was passed through the column, so that adsorbed fractions were eluted. The adsorbed fractions were fractionated by 10 mL. Each fraction was subjected to the SDS-PAGE to check a protein band with the desired molecular weight, and then, the fractions having the protein band were collected. These fractions thus collected were gathered together, which then was subjected to centrifugation at 4°C and 49,000 m/s² for 15 minutes using Amicon (registered trademark) Ultra-15 (Produced by Millipore Corporation), so that it was concentrated to about 20 mL. A second running buffer with the same amount as that of this concentrated solution was added thereto, which then was again subjected to centrifugation using Amicon (registered trademark) Ultra-15 in the same manner as that of the concentration. The second running buffer was added to a concentrated solution thus obtained so that the total amount was 20 mL. The composition of the second running buffer included 50 mmol/L tris-HCl buffer solution (pH7.5), 100 mmol/L potassium chloride, 5 mmol/L EDTA, 5 mmol/L 2-mercaptoethanol, and 10% (w/v) glycerol.

### (3) Gel filtration column chromatography

Next, a gel filtration column (Superdex200 prep grade XK50-65, produced by GE Healthcare) and the high-performance liquid chromatography system were used. The gel filtration column was equilibrated using the second running buffer under the condition where a flow rate was 5 mL/minute. The solution obtained by the above-mentioned affinity column chromatography was applied to the gel filtration column at a flow rate of 1 mL/minute. Thereafter, the second running buffer was passed through the column under the same condition as that of the application, and then, filtered fractions were fractionated by 15 mL. Each fraction was subjected to SDS-PAGE to check a protein band with the desired molecular weight, and then, the fractions having the protein band were collected. These fractions thus collected were gathered together, and the second running buffer was added thereto so that the total amount was 250 mL.

### (4) Strong anion exchange column chromatography

Lastly, a strong anion exchange column (Resource Q (20mL), produced by GE Healthcare) and the high-performance liquid chromatography system were used. The strong anion exchange column was equilibrated using the second running buffer under the condition where a flow rate was 4 mL/minute. The solution obtained by the above-mentioned gel filtration column chromatography was applied to the strong anion exchange column at a flow rate of 1 mL/minute. Thereafter, 120 mL of the second running buffer was passed through the column under the same condition as that of the application, so that the column was washed and non-adsorbed fractions were removed. Subsequently, 420 mL of the second running buffer containing sodium chloride with a concentration gradient from 0 to 300 mmol/L was passed through the column, so that adsorbed fractions were eluted. The adsorbed fractions were fractionated by 10 mL. Each fraction was subjected to SDS-PAGE to check a protein band with the desired molecular weight, and then, the fractions having the protein band were collected. These fractions thus collected were gathered together, which was then subjected to centrifugation at 4°C and 49,000 m/s² for 15 minutes using Amicon (registered trademark) Ultra-15 (produced by Millipore Corporation) so that it was concentrated to about 20 mL. 12 mL of 20 mmol/L tris-HCl buffer solution (pH7.5) was added to this concentrated solution thus obtained, which then was subjected to centrifugation using Amicon (registered trademark) Ultra-15 in the same manner as that of the concentration three times. 20 mmol/L tris-HCl buffer solution (pH7.5) was added to a concentrated solution thus obtained so that the total amount was 5 mL. As described above, purified Aac MutS was obtained. Note here that it was checked that the obtained protein is dimer Aac MutS with the molecular weight of about 96,000 Da.

### [Example 2]

An interaction between Aac MutS and each of various double-stranded DNAs was analyzed.

The analysis of the interaction was conducted using BIACORE 3000 (produced by GE Healthcare) and a BIACORE SA sensor chip (produced by GE Healthcare) according to instructions thereof. The composition of running buffer included 50 mmol/L tris-HCl buffer solution (pH7.6), 50 mmol/L potassium chloride, 0.1 mmol/L EDTA, 20 mmol/L magnesium chloride, and 0.005% Tween (registered trademark) 20. The composition of a renaturation buffer solution used for washing the chip included 1 mol/L sodium chloride and 50 mmol/L sodium hydroxide.

Four types of single-stranded DNAs shown in Table 1 below were provided. In Table 1, C-strand DNA and G-strand DNA are sequences completely complementary to each other. T-strand DNA has the same sequence as that of the C-strand DNA except that a base in the T-strand DNA corresponding to C at 21^{st} base in the C-strand DNA is T. Del-strand DNA has the same sequence as that of the C-strand DNA except that a base in the Del-strand DNA corresponding to C at 21^{st} base in the C-strand DNA has been deleted. As mentioned above, the C-strand DNA and the G-strand DNA are completely complementary to each other (full match), whereas the base in each the T-strand DNA and the Del-strand DNA corresponding to the 21^{st} base of the C-strand DNA has been displaced or deleted. Therefore, each of the T-strand DNA and the Del-strand DNA is a single base mismatch to the G-strand DNA. In this example, hereinafter, the case where a double-stranded DNA between the C-strand DNA and the G-strand DNA that are completely complementary to each other is referred to as "fully-matched", and the case where a double-stranded DNA between the G-strand DNA and the T-strand DNA that is a single base mismatch to the G-strand DNA is referred to as "mismatched", and the case where a double-stranded DNA between the G-strand DNA and the Del-strand DNA in which single base thereof corresponding to the base of G-strand DNA has been deleted is referred to as "deletion".

**[Table 1]**

| |
|---|
| C-strand DNA (SEQ ID NO: 3) |
| 5'-biotin-CCGCTGAATTGCACCGAGCTCGATCCTCGATGATCCTAAGCGATCCATG-3' |
| T-strand DNA(SEQ ID NO: 4) |
| 5'-biotin-CCGCTGAATTGCACCGAGCTTGATCCTCGATGATCCTA.AGCGATCCATG-3' |
| Del-strand DNA (SEQ ID NO: 5) |
| 5'-biotin-CCGCTGAATTGCACCGAGCT=GATCCTCGATGATCCTAAGCGATCCATG-3' |
| G-strand DNA (SEQ ID NO: 6) |
| 5'-CATGGATCGCTTAGGATCATCGAGGATCGAGCTCGGTGCAATTCAGCGG-3' |

The chip was set in BIACORE3000, then the running buffer was passed through a channel of the chip at a flow rate of 10 µL/min, and an experiment was started as follows. First, 5 µmol/L C-strand DNA, 5µmol/L T-strand DNA, and 5 µmol/L Del-strand DNA as regands were passed through the respective three flow cells contained in the chip at a flow rate of 10 µL/min, and they were bound to each other until the Resonance Unit reached about 150 RU (Resonance Unit). Subsequently, 5 µmol/L of the G-strand DNA was injected into each of the flow cells at a flow rate of 20 µL/min for 2 minutes, and thereafter, the flow cells were washed with the running buffer for 10 minutes. Thus, double-stranded DNAs between the C-strand DNA and the G-strand DNA, between T-strand DNA and the same, and between the Del-strand DNA and the same were formed. Then, an Aac MutS solution with the predetermined concentration (0.1, 0.2, 0.5, 1, 2, or 4 µmol/L) was injected into each of the flow cells at a flow rate of 20 µL/min for 10 minutes, and thereafter, the flow cells were washed with the running buffer for 20 minutes. While injecting and washing, signal intensity was measured from the start of the injection of the Aac MutS. In Comparative Example 1, signal intensity was measured by the same treatment as that of Example 2 using Taq MutS derived from *Thermus aquaticus* as substitute for the Aac MutS.

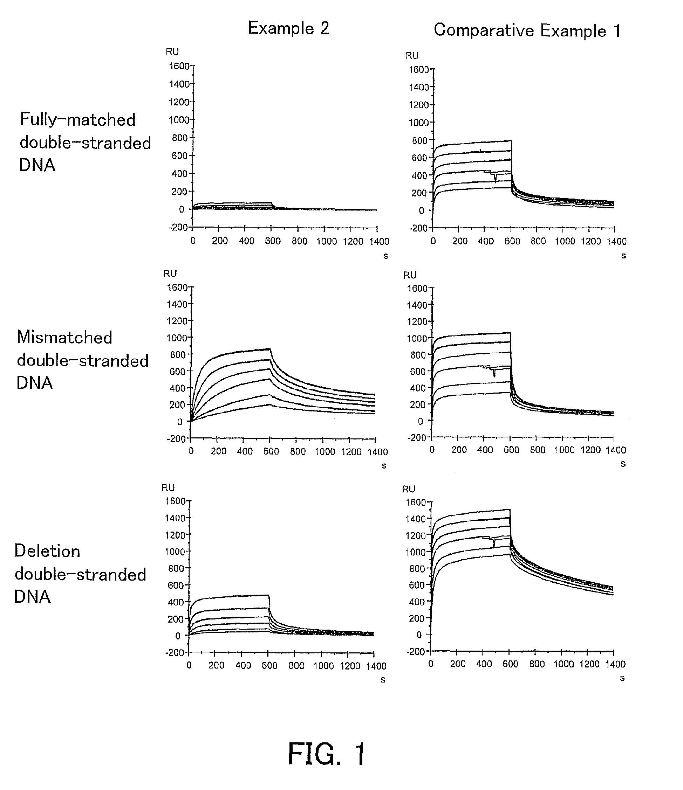

Results of these nucleic acid binding assays were shown in FIG. 1. In graphs shown in FIG. 1, the vertical axis indicates signal intensity (RU) measured by BIACORE, while the horizontal axis indicates an analysis time (second). The results obtained in the time period from 0 to 600 seconds are results obtained in the Aac MutS injecting period, and the results obtained after 600 seconds are results obtained in the washing period. The graphs in a row on the left side of FIG. 1 indicate results of Example 2 using the Aac MutS. The graphs in a row on the right side of FIG. 1 indicate results of Comparative Example 1 using the Taq MutS. In the graphs in the rows on the left side and the right side of FIG. 1, upper graphs indicate the data with respect to the fully-matched double-stranded DNA, middle graphs indicate the data with respect to the mismatched double-stranded DNA, and bottom graphs indicate the data with respect to the deletion double-stranded DNA. Moreover, each of the graphs shows also the results obtained by the use of the MutS having six types of concentrations.

As shown in the graphs in the row on the right side of FIG. 1, with respect to Comparative Example 1 using the Taq MutS, the binding between the mismatched double-stranded DNA and the Taq MutS and the binding between the deletion double-stranded DNA and the same were found. In addition, the binding between the fully-matched double-stranded DNA and the Taq MutS also was found. In contrast, as shown in the graphs in the row on the left side of FIG. 1, with respect to Example 2 using the Aac MutS, the binding between the mismatched double-stranded DNA and the Aac MutS and the binding between the deletion double-stranded DNA and the same were found. However, the binding between the fully-matched double-stranded DNA and the Aac MutS was not found. In addition, it was also found in Comparative Example 1 that the signal was rapidly reduced in the washing period (after 600 seconds), and a dissociation rate between each of the various double-stranded DNAs and the Taq MutS was high. In contrast, it was found out in Example 2 that the signal was not rapidly reduced in the washing period (after 600 seconds), and dissociating between each of the various double strands and the Aac MutS is more difficult than dissociating between each of the various double strands and the Taq MutS in Comparative Example 1. From the above results, it can be said that as compared with the Taq MutS, it is difficult for the Aac MutS to bind to the fully-matched double-stranded DNA, the Aac MutS can specifically bind to the mismatched double-stranded DNA or the deletion double-stranded DNA, it is difficult to dissociate the binding between them, and the binding can be maintained stably.

### [Example 3]

An interaction between Aac MutS and each of various double-stranded DNAs was analyzed in the presence of ADP or ATP.

The signal intensity was measured in the same manner as in Example 2 except that 1 mmol/L ADP or ATP was added to the running buffer of Example 2. The example in the presence of ADP was Example 3-1. The example in the presence of ATP was Example 3-2. In Comparative Example 2, the signal intensity was measured using the Taq MutS in the same manner as in Example 3. The comparative example in the presence of ADP was Comparative Example 2-1, and the comparative example in the presence of ATP was Comparative Example 2-2.

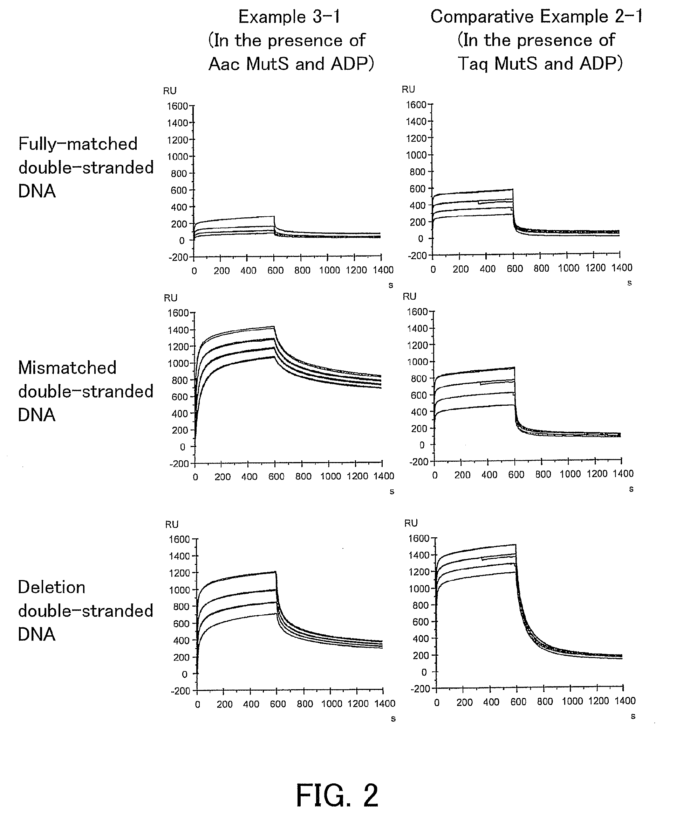

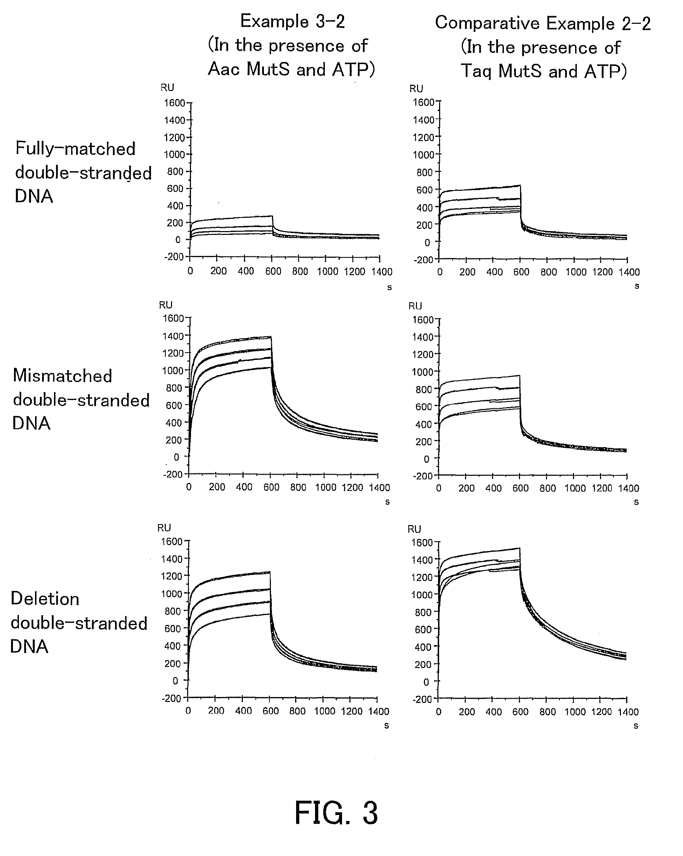

Results of these nucleic acid binding assays are shown in FIGs. 2 and 3. In graphs of Fig. 2 and 3, the vertical axis indicates signal intensity (RU) measured by BIACORE, while the horizontal axis indicates an analysis time (second). The results obtained in the time period from 0 to 600 seconds are results obtained in the Aac MutS injecting period, and the results obtained after 600 seconds are results obtained in the washing period. FIG. 2 shows graphs each indicating the result obtained in the presence of ADP. The graphs in a row on the left side of FIG. 2 indicate the results of Example 3-1 using the Aac MutS, and the graphs in a row on the right side of FIG. 2 indicate the results of Comparative Example 2-1 using the Taq MutS. FIG. 3 indicates the results of Example 3-2 and Comparative Example 2-2 obtained in the presence of ATP. The graphs in a row on the left side of FIG. 3 indicate the results of Example 3-2 using the Aac MutS, and the graphs in a row on the right side of FIG. 3 indicate the results of Comparative Example 2-2 using the Taq MutS. In the graphs in the rows on the left side and the right side of each of FIGs. 2 and 3, upper graphs indicate the data with respect to the fully-matched double-stranded DNA, middle graphs show the data with respect to the mismatched double-stranded DNA, and bottom graphs indicate the data with respect to the deletion double-stranded DNA. Moreover, each of the graphs shows also the results obtained by the use of the MutS having six types of concentrations.

As shown in the graphs in the row on the right side of FIG. 2, the results of Comparative Example 2-1 using the Taq MutS in the presence of ADP were almost the same as those of Comparative Example 1 using the Taq MutS in the presence of no ADP, shown in the graphs in the row on the right side of FIG.1. In contrast, as shown in the graphs in the row on the left side of FIG. 2, in Example 3-1 using the Aac MutS in the presence of ADP, an increase in signal was found in the injecting period (0 to 600 seconds) with respect to the mismatched double-stranded DNA and the deletion double-stranded DNA. This increase was significant as compared with that found in the results of Example 2 using the Aac MutS in the presence of no ADP, shown in the graphs in the row on the left side of FIG. 1. In the washing period (after 600 seconds), the signal with respect to the mismatched double-stranded DNA and the deletion double-stranded DNA in Example 3-1 was decreased very slowly as compared with that with respect to the mismatched double-stranded DNA and the deletion double-stranded DNA in Example 2. From these results, it was found out that, in the presence of ADP, the binding of the Aac MutS to each of the mismatched double-stranded DNA and the deletion double-stranded DNA was accelerated, and the dissociation of the Aac MutS from each of the same was suppressed. In addition, even in the presence of ADP, the binding of the Aac MutS to the fully-matched double-stranded DNA was sufficiently suppressed as in the case of Example 2.

As shown in the graphs in the row on the right side of FIG. 3, the results of Comparative Example 2-2 using the Taq MutS in the presence of ATP were almost the same results as those of Comparative Example 1 using the Taq MutS in the presence of no ATP, shown in the graphs in the row on the right side of FIG.1. In contrast, as shown in the graphs in the row on the left side of FIG. 3, in Example 3-2 using the Aac MutS in the presence of ATP, an increase in signal with respect to the mismatched double-stranded DNA and the deletion double-stranded DNA was found in the injecting period (0 to 600 seconds). This increase was significant as compared with that found in the results of Example 2 using the Aac MutS in the presence of no ATP, shown in the graphs in the row on the left side of FIG. 1. From these results, it was found out that in the presence of ATP, the binding of the Aac MutS to each of the mismatched double-stranded DNA and the deletion double-stranded DNA was accelerated. In addition, even in the presence of ATP, the binding of the Aac MutS to the fully-matched double-stranded DNA was suppressed sufficiently as in the case of Example 2.

The dissociation constant of each MutS and each double-stranded DNA was determined from these results obtained by nucleic acid binding assay. These results are shown in Table 2 below. In Table 2, K_{D(full)} indicates the dissociation constant between each MutS and the fully-matched double-stranded DNA, K_{D(mis)} indicates the dissociation constant between each MutS and the mismatched double-stranded DNA, and K_{D(full)}/KD₍ₘᵢₛ₎ indicates the ratio between them.

**[Table 2]**

| Aac MutS | | | | |
|---|---|---|---|---|
| ADP | | K_{D(full)} (mol/L) | K_{D(miss)} (mol/L) | K_{D(ull)}/ K_{D(mis)} |
| Example 2 | - | 6.55 × 10⁻⁵ | 1.45 × 10⁻⁶ | 45.17 |
| Example 3-1 | ADP | 1.98 × 10⁻⁵ | 3.30 × 10⁻⁷ | 60.00 |

| Taq MutS | | | | |
|---|---|---|---|---|
| ADP | | K_{D(full)} (mol/L) | K_{D(mis)} (mol/L) | K_{D(full)}/ K_{D(mis)} |
| Comp. Ex. 1 | - | 2.27 × 10⁻⁶ | 6.22 × 10⁻⁷ | 3.59 |
| Comp. Ex. 2-1 | ADP | 6.58x10-6 | 2.26 × 10⁻⁶ | 2.91 |

As shown in Table 2, a change in K_{D(full)}/K_{D(mis)} between Comparative Examples 1 and 2-1 using the Taq MutS due to the addition ofADP was slight. In contrast, K_{D(full)}/K_{D(mis)} was increased from about 45 of Example 2 using the Aac MutS to about 60 of Example 3-1 using the same by adding ADP. Thus, it was proved in the reaction kinetics that ADP can suppress the dissociation between the Aac MutS and the mismatched double-stranded
DNA.

### [Example 4]

An interaction between Aac MutS and each of various double-stranded DNAs was analyzed by a gel shift assay using electrophoresis.

A fully-matched double-stranded DNA between C-strand DNA and G-strand DNA and a mismatched double-stranded DNA between T-strand DNA and the G-strand DNA were produced by a method described below using the same single-stranded DNAs as those used in Example 2. First, the 2 µmol/L single-stranded DNAs were mixed according to each combination, whereby a DNA solution was obtained. The DNA solution was heated at 95°C for 10 minutes, so that it was completely degenerated. The DNA solution after the heating was cooled to 30°C at 0.1 °C/second. Thus, each of the double-stranded DNAs was generated. 2.5 uL of the DNA solution after the cooling was mixed with 2.5 uL of 4 × binding buffer solution, and Aac MutS further was added thereto. Then, ADP or ATP and sterile water were added to this mixed solution thus obtained immediately before an incubation so that the total amount thereof became 10 µL, which was then incubated at 60°C for 30 minutes. The final concentration of the Aac MutS was set to 0, 1, 2, or 4 µmol/L, and that of ADP or ATP was 0 or 1 mmol/L. The composition of the 4 × binding buffer solution included a 200 mmol/L tris-HCl buffer solution (60°C, pH 7.6), 200 mmol/L potassium acetate, 80 mmol/L magnesium chloride, 0.4 mmol/L EDTA, 5 mmol/L 2-mercaptoethanol, and 40% glycerol. After the incubation of this solution at 60°C for 30 minutes, 2 uL of 6 × Loading Dye was added thereto, and then the solution was subjected to an electrophoresis using 6% polyacrylamide gel. The electrophoresis was conducted in a 1 × TAE buffer solution containing 20 mmol/L magnesium acetate at 4°C, 45 mA, and 100V for 100 minutes. A gel obtained after the electrophoresis was immersed in a staining solution (SYBR (registered trademark) Green I, produced by Lonza Group Ltd.) for 30 minutes so that the gel was stained. Then, DNA was detected using transmitted UV light. In Comparative Example 3, a gel shift assay was conducted in the same manner as in Example 4 except that the Taq MutS was used as substitute for the Aac MutS. The final concentration of the Taq MutS was 1 µmol/L.

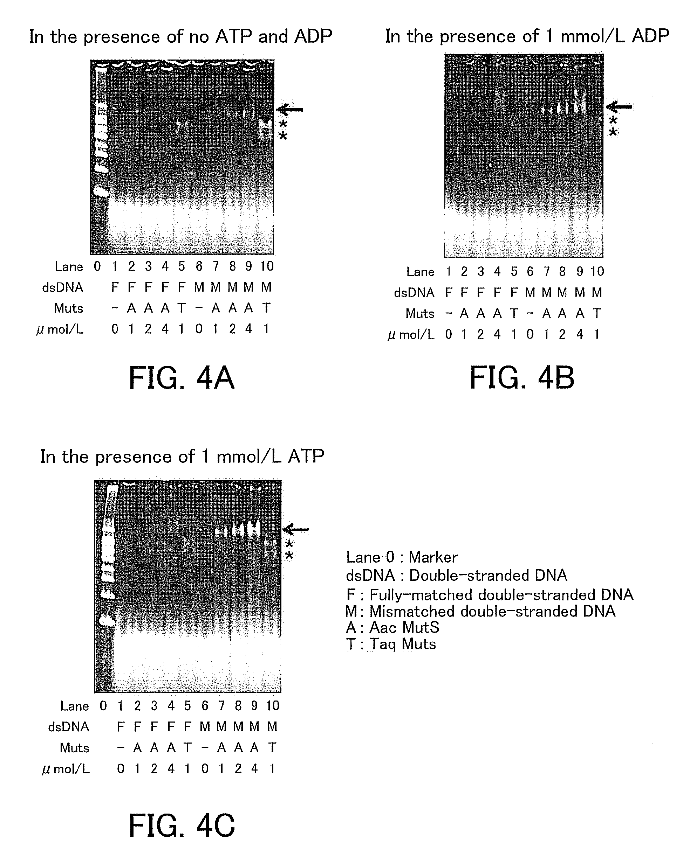

These results are shown in FIG. 4. FIG. 4A shows an electrophoretogram indicating a result obtained by conducting a gel shift assay in the presence of no ATP and ADP FIG. 4B shows an electrophoretogram indicating a result obtained by conducting a gel shift assay in the presence of 1 mmol/LADP. FIG. 4C shows an electrophoretogram indicating a result obtained by conducting a gel shift assay in the presence of 1 mmol/LATP. In FIGs. 4A and 4C, a lane 0 indicates a marker (100bp DNA Ladder (product name), produced by TAKARA BIO INC.) of the electrophoresis. In FIGs. 4A to 4C, lanes 1 to 5 indicate results with respect to the fully-matched double-stranded DNA, and lanes 6 to 10 indicate results with respect to the mismatched double-stranded DNA. The lanes 1 and 6 indicate results of Example 4 using 0 µmol/L Aac MutS. The lanes 2 and 7 indicate results of Example 4 using 1 µmol/L Aac MutS. The lanes 3 and 8 indicate results of Example 4 using 2 µmol/L Aac MutS. The lanes 4 and 9 indicate results of Example 4 using 4 µmol/L of Aac MutS. The lanes 5 and 10 indicate results of Comparative Example 3 using 1 µmol/L Taq MutS. An arrow indicates a band of gel shift caused by a binding between the Aac MutS and the double-stranded DNA in Example 4. A symbol * indicates a band of gel shift caused by a binding between the Taq MutS and the double-stranded DNA in Comparative Example 3.

As shown in the lanes 5 and 10 of each of FIGs. 4A to 4C, bands were found at the respective positions each indicated by * regardless of the presence or absence of ATP or ADP in Comparative Example 3 using the Taq MutS. Thus, the Taq MutS bound to each of the fully-matched double-stranded DNA and the mismatched double-stranded DNA at the same degree as each other. The degree of the binding was not changed according to the addition of ATP or ADP. In contrast, as shown in the lanes 1 to 4 and 6 to 9 of FIG. 4A, bands were found at the positions each indicated by the arrow in Example 4 using the Aac MutS in the presence of no ATP and ADP. Therefore, the Aac MutS bound to the mismatched double-stranded DNA. Further, as shown in the same, no band was found at the positions each indicated by the arrow in the same. Therefore, the Aac MutS slightly bound to the fully-matched double-stranded DNA. Moreover, as shown in the lanes 6 to 9 of FIGs. 4B and 4C, color of the bands at the positions each indicated by the arrow were intense in the presence of ADP or ATP Thus, it was found out that the binding between the Aac MutS and the mismatched double-stranded DNA was significantly accelerated.

### [Example 5]

A DNA amplification reaction was conducted by the Smart Amplification Process method in the presence of Aac MutS, and a single base mutation (at -3826 position) in the UCP1 gene was analyzed on the basis of the presence or absence of amplification.

**[Table 3]**

| (Amplification reaction solution) | |
|---|---|
| 2 × Reaction buffer solution | 12.5 µL |
| Genomic DNA solution | 1.0 µL |
| Aac DNA polymerase solution | 1.0 µL |
| MutS solution (predetermined concentration) | 1.0 µL |
| 10 mmol/LADP solution | 2.5 µL |
| Primer mixed solution | 1.25 µL |
| Sterile water | 5.75 µL |
| Total | 25.0 µL |

**[Table 4]**

| (2 × Reaction buffer solution) | |
|---|---|
| 20 mmol/L | Tris-HCl buffer solution (25°C, pH 8.6) |
| 50 mmol/L | Potassium acetate |
| 10 mmol/L | Ammonium sulfate |
| 8 mmol/L | Magnesium sulfate |
| 5% | DMSO |
| 0.1% | Tween (registered trademark) 20 |
| 1.4 mmol/L | dNTP |

**[Table 5]**

| (Aac DNA polymerase solution) | |
|---|---|
| 12 unit/µL | Aac DNA polymerase |
| 10 mmol/L | Tris-HCl buffer solution (25°C, pH 8.0) |
| 50 mmol/L | Potassium chloride |
| 0.1 mmol/L | EDTA |
| 1 mmol/L | DTT |
| 0.1% | Triton (registered trademark) X-100 |
| 50% | Glycerol |

A MutS solution in the composition of the reaction solution was prepared using a buffer solution for preparing MutS below so that the concentration thereof was the predetermined concentration (0, 10, 11, or 12 µg/µL). Note here that the amount of the MutS in 25 µL of the reaction solution was 0, 10, 11, or 12 µg.

**[Table 6]**

| | |
|---|---|
| (Buffer solution for preparing MutS) | |
| 20 mmol/L | Tris-HCl buffer solution (25°C, pH 7.5) |
| 100 mmol/L | Potassium chloride |
| 0.1 mmol/L | EDTA |
| 1 mmol/L | DTT |
| 0.1% | Triton (registered trademark) X-100 |
| 50% | Glycerol |

A primer mixed solution in the composition of the reaction solution was prepared by mixing the 100 µmol/L primers shown below so that the volume ratio TP : FP : BP : OPF : OPR thereof became 8 : 8 : 4 : 1 : 1. As the TP, any of TP WT and TP MT was used. The TP WT and the TP MT are target primers that can hybridize to the region including a detection site in the UCP1 gene. In the TP WT, underlined A is of a wild type, and in the TP MT, underlined G is of a mutant type. Hereinafter, the following primer set containing the TP WT is referred to as a wild-type primer set, and the following primer set containing the TP MT is referred to as a mutant-type primer set.
UCP1 TP WT (SEQ ID NO: 7)
5'-CAAGTGCATTTATGTAACAAATTCTCCTTTCCTTT-3'
UCP1 TP MT (SEQ ID NO: 8)
5'-CGAGTGCATTTATGTAACAAATTCTCCTTTCCTTT-3'
UCP1 FP (SEQ ID NO: 9)
5'-TTTATATATATATAAAGCAGCGATTTCTGATTGACCA-3'
UCP1 BP (SEQ ID NO: 10)
5'-TAATGTGTTCTACATTTT-3'
UCP1 OPF (SEQ ID NO: 11)
5'-GATTTTTATTTAATAGGAAGACATT-3'
UCP1 OPR (SEQ ID NO: 12)
5'-GACGTAGCAAAGGAGTGGCAGCAAG-3'

As a template DNA, human genomic DNA in which a sequence of the UCP1 gene is of a wild type (a base at -3826 position is A) or of a mutant type (a base at -3826 position is G) was used. The genomic DNA was diluted by a TE buffer solution so that the concentration thereof became 13.3 ng/µL.
This genomic DNA solution thus obtained was subjected to a heat treatment at 98°C for 3 minutes and then was quickly cooled on ice. An amplification reaction solution having the above-described composition was prepared on ice, and this reaction solution thus obtained was incubated at 60°C for 120 minutes. The generation of amplification product was monitored using a real-time fluorescence device (Mx3000P (product name), produced by Stratagene). In Example 5, Aac MutS was used as the MutS.

On the other hand, in Comparative Example 4, monitoring was conducted in the same manner as in Example 5 except that Taq MutS was used as the MutS, and the composition of the reaction solution included no ADP solution and included 8.25 µL of sterile water was included. The concentration of the MutS in the MutS solution was set to the predetermined concentration (4, 5, 6, or 7 µg/µL). Thus, the amount of the Taq MutS in the 25 µL of the reaction solution was 4, 5, 6, or 7 µg.

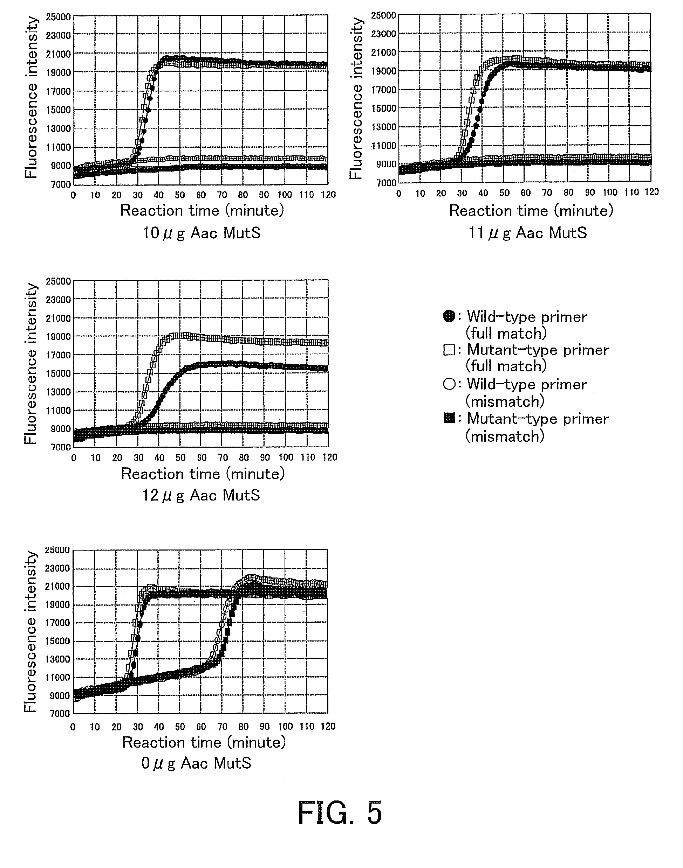

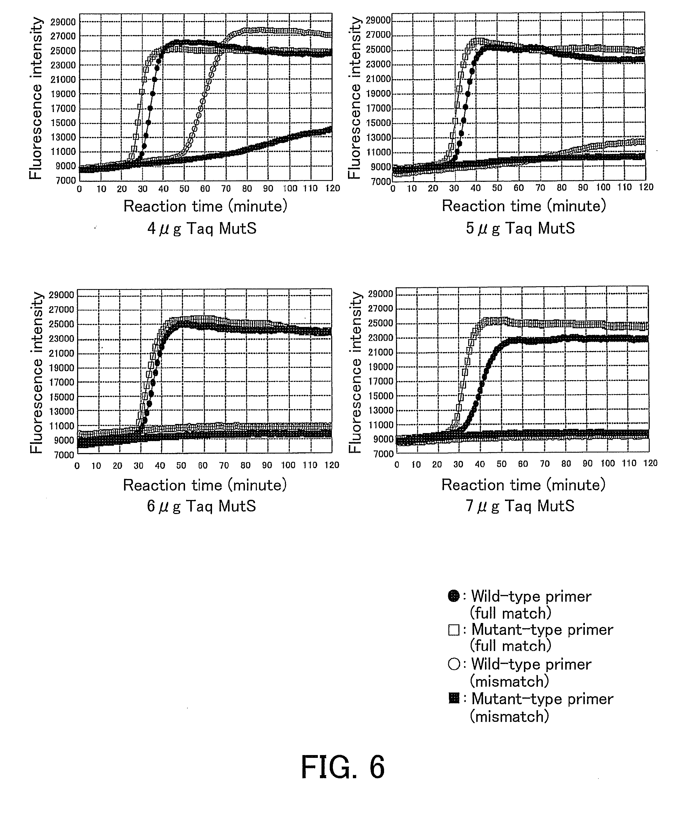

These results are shown in FIGs. 5 and 6. FIGs. 5 and 6 show graphs each indicating a relationship between the reaction time (minute) and fluorescence intensity obtained by real-time monitoring of amplification.
The amount of the MutS in 25 µL of the reaction solution was described in each of the graphs. FIG. 5 shows results of Example 5 using the Aac MutS, and FIG. 6 shows results of Comparative Example 4 using the Taq MutS. In FIGs. 5 and 6, the vertical axis indicates fluorescence intensity (FU: Fluorescence Unit), while the horizontal axis indicates the reaction time (minute). In addition, the graphs of FIGs. 5 and 6 also show results obtained by using combinations between a wild-type genomic DNA and a wild-type primer set (●), between a wild-type genome DNA and a mutant-type primer set (■), between a mutant-type genomic DNA and a mutant-type primer set (□), and between a mutant-type genomic DNA and a wild-type primer set (○). The combinations between a wild-type genomic DNA and a wild-type primer set and between a mutant-type genomic DNA and a mutant-type primer set are combinations each forming the fully-matched double-stranded DNA. The combinations between a wild-type genomic DNA and a mutant-type primer set and between a mutant-type genomic DNA and a wild-type primer set are combinations each forming the mismatched double-stranded DNA.

As shown in FIG. 6, in Comparative Example 4 using the Taq MutS, amplification with respect to the combinations (o and ■) each forming the mismatched double-stranded DNA was suppressed, and amplification with respect to the combinations (● and □) each forming the fully-matched double-stranded DNA was not inhibited, only under the condition where the amount of the Taq MutS was in the range from 6 to 7 µg in 25 µL of the reaction solution. However, when the amount of the Taq MutS was 4 µg, which is out of the range of this condition, amplification with respect to the combinations (o and ■) each forming the mismatched double strand was found. Further, when the amount of the Taq MutS was 5 µg, which is out of the range of this condition, amplification with respect to the combination (o) forming the mismatched double strand was found. From these results, the effective concentration range of the Taq MutS was extremely narrow. In contrast, as shown in FIG.5, in Example 5 using the Aac MutS, amplification with respect to the combinations (o and ■) each forming the mismatched double-stranded DNA was suppressed, and amplification with respect to the combinations (● and □) each forming the fully-matched double-stranded DNA was not inhibited, even under the condition where the amount of the Aac MutS was in the range from 10 to 12 µg in 25 µL of the reaction solution. From these results, it was found out that the effective concentration range of the Aac MutS was wider than that of the Taq MutS.

### [Example 6]

A DNA amplification reaction was conducted by the Smart Amplification Process method in the presence of the Aac MutS and the Taq MutS, and a single base mutation (at -3826 position) was analyzed on the basis of the presence or absence of amplification.

The amplification was monitored in the same manner as in Example 5 except that a MutS solution containing the Aac MutS and the Taq MutS was used as substitute for the MutS solution containing only the Aac MutS. The amounts of the Aac MutS and the Taq MutS protein in 25 µL of the reaction solution are shown below. In Example 6-1, the total amount of the Aac Mutes and the Taq Mutes in 25 µL of the reaction solution was set to 7 µg. In Example 6-2, the amount of the Aac MutS in 25 µL of the reaction solution was set to the same amount as that of the Taq MutS in the same.

**[Table 7]**

| (Example 6-1) | | | | |
|---|---|---|---|---|
| Aac Muts (µg) | 2 | 3 | 4 | 5 |
| Taq Muts (µg) | 5 | 4 | 3 | 2 |

| (Example 6-2) | | | | |
|---|---|---|---|---|
| Aac Muts (µg) | 4 | 5 | | |
| Taq MutS (µg) | 4 | 5 | | |

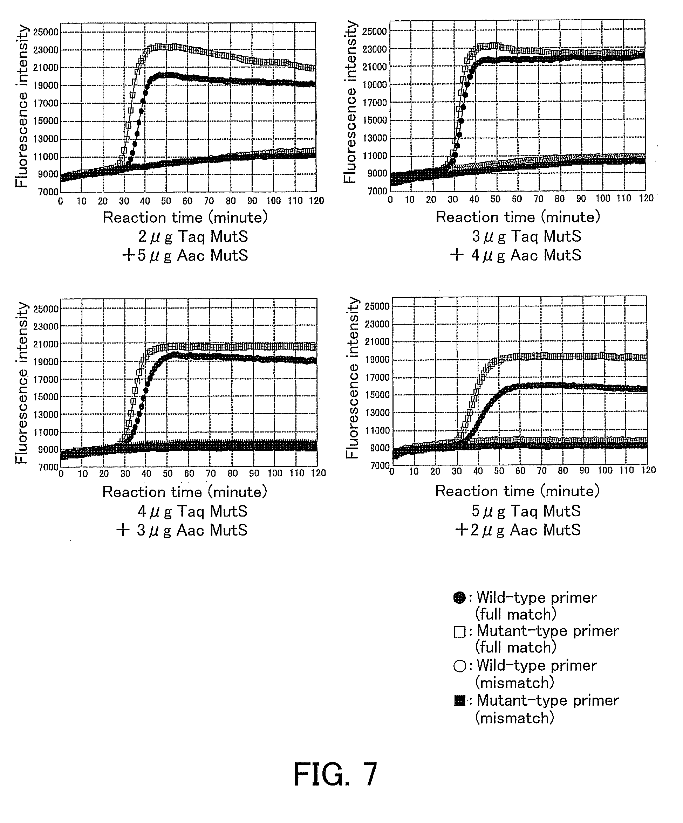

These results are shown in FIGs. 7 and 8. FIGs. 7 and 8 show graphs each indicating a relationship between the reaction time (minute) and fluorescence intensity obtained by real-time monitoring of amplification. The amount of the MutS in 25 µL of the reaction solution was described in each of the graphs. FIG. 7 shows results of Example 6-1 in the case where the total amount of the Aac MutS and the Taq MutS was 7 µg. FIG. 8 shows results of Example 6-2 in the case where the amount of the Aac MutS was the same as that of the Taq MutS. Explanations of the graphs of FIGs. 7 and 8 are the same as those for FIGs. 5 and 6.

As shown in FIG. 7, in Example 6-1, amplification with respect to the combinations each forming the mismatched double-stranded DNA was suppressed, and amplification with respect to the combinations each forming the fully-matched double-stranded DNA was not inhibited, even though the total amount of the MutS in 25 µL of the reaction solution was set to 7 µg, and the ratio between the Aac MutS and the Taq MutS was changed from 2 : 5 to 5 : 2. From this result, it was found out that it is possible to use the Aac MutS in combination with Taq MutS. In addition, it also was found out that, by the use of the Aac MutS and Taq MutS in combination, the amount of the Aac MutS to be used can be reduced, and both the Aac MutS and the Taq MutS can be used in the wide effective range.

Moreover, as shown in FIG. 8, in Example 6-2, amplification with respect to the combinations each forming the mismatched double-stranded DNA was suppressed, and amplification with respect to the combinations each forming the fully-matched double-stranded DNA was not inhibited, even though the amount of the Taq MutS in 25 µL of the reaction solution was set to the same as that of the Aac MutS in the same, and the total amount of the MutS was set in the range from 8 to 10 µg. From this result, it was found out that it is possible to use the Aac MutS in combination with Taq MutS. In addition, it was also found out that, by the use of the Aac MutS and Taq MutS in combination, the amount of the Aac MutS to be used can be reduced, and both the Aac MutS and the Taq MutS can be used in the wide effective range.

From these reasons, it was found out that functions of the MutS can be exerted in the wide concentration range by changing the ratio between and the total amount of the Aac MutS and the Taq MutS in the reaction solution.

### Industrial Applicability

As described above, the Aac MutS protein of the present invention can specifically recognize and bind to a double-stranded nucleic acid having a mismatched base pair, for example. Therefore, when the Aac MutS of the present invention is used for amplification of a target sequence including a target site, the Aac MutS binds specifically to a mismatched base pair, so that an extension from a primer can be suppressed effectively. Thus, according to the determination method of the present invention using the Aac MutS of the present invention, the presence or absence of a mutation can be determined on the basis of the presence or absence of amplification with high accuracy. Therefore, it can be said that the Aac MutS and the determination method of the present invention are very useful tools in the fields of gene analyses, for example.

## Claims

1. A novel MutS protein comprising: an amino acid sequence (A) or (B) below:
(A) an amino acid sequence shown in SEQ ID NO: 2; and
(B) an amino acid sequence that is obtained by deletion, displacement, insertion, or addition of one or several amino acids in the amino acid sequence (A) and is of a protein having a binding activity to a mismatched base pair contained in a double-stranded nucleic acid.

2. The novel MutS protein according to claim 1, wherein the protein is derived from genus *Alicyclobacillius*.

3. The novel MutS protein according to claim 2, wherein the protein is derived from *Alicyclobacillus acidocaldarius*.

4. A nucleic acid that encodes a novel MutS protein, the nucleic acid comprising: any of nucleic acids (a) to (f) below:
(a) a nucleic acid having a base sequence of SEQ ID NO: 1;
(b) a nucleic acid that hybridizes to the nucleic acid (a) under stringent conditions and encodes a protein having a binding activity to a mismatched base pair contained in a double-stranded nucleic acid;
(c) a nucleic acid that has a base sequence having a homology of 80% or more to a base sequence of the nucleic acid (a) and encodes a protein having a binding activity to a mismatched base pair contained in a double-stranded nucleic acid;
(d) a nucleic acid that has a base sequence obtained by deletion, displacement, insertion, or addition of one or several bases in the base sequence of the nucleic acid (a) and encodes a protein having a binding activity to a mismatched base pair contained in a double-stranded nucleic acid;
(e) a nucleic acid that encodes a protein having an amino acid sequence shown in SEQ ID NO: 2; and
(f) a nucleic acid that has an amino acid sequence obtained by deletion, displacement, insertion, or addition of one or several amino acids in the amino acid sequence shown in SEQ ID NO: 2 and encodes a protein having a binding activity to a mismatched base pair contained in a double-stranded nucleic acid.

5. A recombinant vector comprising the nucleic acid according to claim 4.

6. A transformant comprising the recombinant vector according to claim 5.

7. A production method for producing the novel MutS protein according to claim 1, the production method comprising:
culturing the transformant according to claim 6.

8. A determination method for determining a presence or absence of a mutation at a target site in a sample nucleic acid, the determination method comprising the step (1) or (I'), and the step (II) below:
(I) the step of amplifying a target sequence including the target site contained in the sample nucleic acid using a primer that can hybridize to a region including the target site contained in the sample nucleic acid in a presence of the novel MutS protein according to claim 1;
(I') the step of amplifying a target sequence including the target site contained in the sample nucleic acid using a primer for amplifying the sample nucleic acid in a presence of the novel MutS protein according to claim 1 and a probe that can hybridize to the region including the target site contained in the sample nucleic acid; and
(II) the step of checking for presence or absence of amplification.

9. The determination method according to claim 8, wherein
in the step (I) or (I'), the target sequence is amplified in a coexistence of the novel MutS protein and at least one additive selected from the group consisting of additives of ADP, ATP, and derivatives thereof.

10. The determination method according to claim 9, wherein
a concentration of the additive in a reaction solution used for an amplification reaction is in a range from 0.01 to 100 mmol/L.

11. The determination method according to claim 8, wherein
an amount of the novel MutS protein is in a range from 0.01 to 1000 µg per 25 µL of a reaction solution used for an amplification reaction.

12. The determination method according to claim 8, wherein
the target sequence is amplified in a coexistence of the novel MutS protein and a MutS protein derived from genus *Thermus.*

13. The determination method according to claim 12, wherein
the MutS protein derived from genus *Thermus* is a MutS protein derived from *Thermus aquaticus*.

14. The determination method according to claim 12, wherein
a ratio (weight ratio (A: T)) of the MutS protein derived from genus *Thermus* (T) to be added with respect to the novel MutS protein (A) is in a range from 1 : 0.05 to 1 : 50.

15. The determination method according to claim 12, wherein, per 25 µl of a reaction solution used for an amplification reaction,
an amount of the novel MutS protein is in a range from 0.01 to 1000 µg
an amount of the MutS protein derived from genus *Thermus* is in a range from 0.01 to 1000 µg,
a total amount of the novel MutS protein and the MutS protein derived from genus *Thermus is* in a range from 0.02 to 2000 µg.

16. The determination method according to claim 8, wherein
in the step (I), a primer that can hybridize to the region in which a base at the target site is of a mutant type is used, or in the step (I'), a probe that can hybridize to the region in which the base at the target site is of a mutant type is used, and
when amplification is found in the step (II), it is determined that the base at the target site is of a mutant type, and when amplification is not found in the same, it is determined that the base at the target site is of a wild type.

17. The determination method according to claim 8, wherein
in the step (I), a primer that can hybridize to the region in which a base at the target site is of a wild type is used, or in the step (I'), a probe that can hybridize to the region in which the base at the target site is of a wild type is used,
when amplification is found in the step (II), it is determined that the base at the target site is of a wild type, and when amplification is not found in the same, it is determined that the base at the target site is of a mutant type.

18. The determination method according to claim 8, wherein
a polymerase is used for amplifying the target site, and
the polymerase is derived from genus *Alicyclobacillus.*

19. The determination method according to claim 18, wherein
the polymerase is derived from *Alicyclobacillus acidocaldarius.*

20. The determination method according to claim 18, wherein
the polymerase has a strand displacement ability.

21. The determination method according to claim 8, wherein
an amplification reaction is conducted while changing a temperature.

22. The determination method according to claim 21, wherein
the amplification reaction is a polymerase chain reaction.

23. The determination method according to claim 8, wherein
the amplification reaction is conducted at a constant temperature.

24. The determination method according to claim 23, wherein
the amplification reaction is at least one selected from the group consisting of an SDA method, an improved SDA method, an NASBA method, a LAMP method, an ICAN method, a self-sustained sequence replication method, a TMA method, a Q-beta replicase method, a Smart Amplification Process method, an Invader method, and an RCA method.
